# EUROPEAN PATENT APPLICATION

(11) **EP 2 857 915 A1**
(43) Date of publication of application: **08.04.2015**
(21) Application number: 13801196.0
(22) Date of filing: 31.05.2013
(51) Int. Cl.: G05B 19/418, C12N 1/00, C12Q 1/04, G06Q 50/04

(54) **CULTURE-MEDIUM INFORMATION REGISTRATION SYSTEM, COMMUNICATION TERMINAL, PROGRAM, HEALTH MANAGEMENT SYSTEM, AND FILM-TYPE CULTURE MEDIUM**

(30) Priority: 04.06.2012 JP 2012127595
(71) Applicant: Dai Nippon Printing Co., Ltd., Tokyo 162-8001 (JP)
(72) Inventor: KAGOTA, Masanori, Tokyo 162-8001 (JP); BISE, Ryoma, Tokyo 162-8001 (JP); TSUZUKI, Yuji, Tokyo 162-8001 (JP); SAITOU, Rui, Tokyo 162-8001 (JP); KYOUTANI, Hitoshi, Tokyo 162-8001 (JP)
(74) Representative: Beck Greener
(86) International application number: PCT/JP2013/065219
(87) International publication number: WO 2013/183562

(57) **Abstract**

The sanitary management system extracts food produced or inspected using a work line including one or more production process or inspection process as a specimen, and registers culture medium information obtained by a portable communication terminal device before and after culturing the extracted specimen to a server device in correspondence with work line related information related to the work line including information of sanitary management of the work line from which the specimen is extracted.

## Description

### TECHNICAL FIELD

The present invention relates to a sanitary management system for food.

### BACKGROUND TECHNIQUE

Recently, due to growth of the food service industry such as fast food and increase of easy processed food, opportunity of eating food cooked by others is remarkably increasing. On the other hand, if dangerous substance such as toxic substance or microbe is mixed into foodstuffs such as processed ingredients or processed food, it directly affects the human body. Therefore, in handing the processed food, it is required to strictly manage not only safety of raw material but also sanitation of production process to ensure the safety of food through the whole food processing.

Particularly, beginning from food poisoning cases problematically occurring in various regions, it is a proposition to further improve the safety of food. There are increasing number of companies not only complying with the food sanitation law but also introducing "HACCP (Hazard Analysis Critical Control Point System)" and/or "FSSC (Food Safety System Certification 22000)".

In this situation, for example, as a system capable of performing sanitary management and safety management in the production process of processed food, there is known a food production information collecting and managing system which collects food production information such as a temperature history through the whole production processing of food and used facilities and which automatically records it into a recording medium to output total management information (for example, Patent Reference 1). Similarly, there is known a food manufacturing history information management system which manages manufacturing history information of products including sanitary information of workers (for example, Patent Reference 2).

On the other hand, as a method of detecting microbes mixed in processed food, there is known a method of using a film-type culture medium and counting specimens cultured on the film-type culture medium based on its image (for example, Patent Reference 3).

### PRIOR ART REFERENCES

### PATENT REFERENCES

Patent Reference 1: Japanese Patent Application Laid-Open under No. 11-160160
Patent Reference 2: Japanese Patent Application Laid-Open under No. 2005-107563
Patent Reference 3: Japanese Patent Application Laid-Open under No. 2001-212013

### SUMMARY OF INVENTION

### PROBLEM TO BE SOLVED BY THE INVENTION

However, in the Patent References 1 and 2 mentioned above, the temperature history or the workers in the production process is merely managed in association with a worker ID, production facility information and product information, and those references do not disclose registering various information at the time of performing sanitary management based on food serving as a specimen extracted from the production process.

Additionally, in Patent Reference 3 mentioned above, the sanitary management of the production process can be performed by applying the disclosed method. However, since it is necessary to accurately manage plural specimens when the method is applied to the sanitary management of the production process, it forces the worker complicated works and may induce error by the worker. Therefore, itisdifficultto easily and accurately perform sanitary management by the method of Patent Reference 3.

The present invention is achieved to solve the problem described above, and its object is to provide a culture medium information registration system capable of solving complicated work by a worker who registers culture medium information related to a culture medium to prevent registration error of the culture medium information and other information and registering various information including the culture medium information whose data management can be easily performed.

### MEANS FOR SOLVING THE PROBLEM

(1) In order to solve the above problem, there is provided a culture medium information registration system which cooperates with a communication terminal device to register, with a database, culture medium information indicating information of a culture medium on which food produced or inspected by a work line including one or more production process or inspection process is cultured as a specimen, the system comprising: a culture medium identification information obtaining unit which cooperates with the communication terminal device to obtain culture medium identification information for identification from other culture media; a work identification information obtaining unit which obtains work identification information which is inputted by the communication terminal device and which identifies a work of the work line; a search unit which searches line related information related to the work line based on the obtained work identification information, the line related information including information of sanitary management of the work line stored in the database for each work identification information; and a registration unit which registers the culture medium information including at least the obtained culture medium identification information, with the database, in correspondence with the searched line related information.

With this configuration, since the culture medium information registration system according to the present invention can cooperate with the communication terminal device such as a portable communication terminal device to register the culture medium identification information with the database in correspondence with the line related information, the culture medium information and the line related information may be easily registered in correspondence with each other, in comparison with the conventional case where the line related information necessary to produce a report of the film-type culture medium is directly and manually entered by a worker in a predetermined space of a film-type culture medium or a container of the culture medium.

Therefore, the culture medium information registration system according to the present invention can eliminate the complicated work of registering each information to prevent the registration error, and can register the culture medium information in such a manner that the data management becomes easy.

Particularly, in the culture medium information registration system according to the present invention, the registration error can be remarkably reduced by registering the culture medium information with the database and the difficult management of the culture medium becomes easy, thereby saving the resources without wastefully consuming the culture medium. The culture medium information registration system according to the present invention can analyze the sanitary management related to the work line by the electronic data, and supplying the analysis result can be electronically executed. Therefore, resources can be saved in comparison with the case where various data analysis and its supply are performed by using physical resource such as a paper medium.
(2) Also, the culture medium information registration system according to the present invention may further comprise an image data obtaining unit which obtains image data of the specimen imaged by the communication terminal device, and the registration unit registers the culture medium information including at least the obtained image data and the obtained culture medium identification information, with the database, in correspondence with the searched line related information.
   With this configuration, since the culture medium information registration system according to the present invention can register the image data of the culture medium with the database in correspondence with the culture medium identification information and the line related information, the number of colonies can be detected by the predetermined image analysis of the image data and its information can be registered, for example.
   Therefore, since the culture medium information registration system according to the present invention can automate the analysis of each culture medium for registration, it is possible to easily register the information enabling not only the evaluation of the specimen cultured in each culture medium but the automatic determination of the sanitary condition of the process, the work line or the lot.
(3) Also, the culture medium information registration system according to the present invention may further comprise a time information obtaining unit which cooperates with the communication terminal device to obtain time information indicating at least one of a start time of starting culture of the specimen in the culture medium and an imaging time of imaging the image data.
   With this configuration, since the culture medium information registration system according to the present invention can register the start time of the culture of the specimen or the imaging time of the image data with the database in correspondence with the culture medium identification information and the line related information, the culture medium varying with the passage of time, i.e., at the culture start time, 24 hours, 48 hours or more from the start of the culture can be registered with the database together with the image data.
   Accordingly, when the culture condition of the specimen is inspected at different time in a single culture medium, the culture medium information registration system according to the present invention can prevent the erroneous registration of the culture medium information, can easily manage the complicated information of the culture medium, and can register the culture medium information such that the culture medium information can be easily confirmed in time series after the registration.
(4) Also, in the culture medium information registration system according the present invention, the culture medium identification information obtaining unit obtains the culture medium identification information by obtaining the imaged culture medium identification information included in the image data and recognizing the imaged culture medium identification information by image analysis.
   With this configuration, in the culture medium information registration system according to the present invention, if the culture medium identification information is formed as a barcode such as a two-dimensional barcode or the characters at the container, the packaging material, a blank space of the marking for management or other parts for managing the culture medium itself, the culture medium identification information imaged together with the culture medium can be recognized at the time of imaging the culture medium. Thus, the input operation by the worker can be simplified, and the culture medium information can be registered with identifying it from other culture medium information.
   Therefore, the culture medium information registration system according to the present invention can register the culture medium information and the line related information easily in correspondence with each other, eliminate the complicated work of registering each information to prevent the registration error, and register the culture medium information in such a manner that the data management becomes easy.
(5) Also, the culture medium information registration system according to the present invention may further comprise a detection unit which detects a number of colonies in the specimen included in the image data based on the obtained image data, and the registration unit registers the number of colonies with the database, as the culture medium information, in correspondence with the line related information.
   With this configuration, since the culture medium information registration system according to the present invention can detect the number of colonies in the culture medium and register it with the culture medium information in correspondence with the line related information, the analysis result of the culture medium can be automatically registered by imaging the culture medium to which the identification information is attached.
(6) Also, the culture medium information registration system according to the present invention may further comprise a process information obtaining unit which cooperates with the communication terminal device to specify at least one of a process of extracting the specimen from the work line and an extraction area of extracting the specimen and obtain the specified information as the culture medium information.
   With this configuration, since the culture medium information registration system according to the present invention can register the information specifying the position on the work line of the specimen cultured in the culture medium or the process in which the specimen is extracted, not only the information used for the evaluation of the specimen but also the information enabling automatic determination of the sanitary condition of the work line can be easily registered.
(7) Also, in the culture medium information registration system according to the present invention, the line related information includes at least one of line information for specifying the work line, process information for specifying the process of extracting the specimen, lot information for specifying a lot including the specimen, a specimen type indicating a type of the specimen, a culture start time of the specimen, and a dilution rate for culturing the specimen in the culture medium.
   With this configuration, since the culture medium information registration system according to the present invention can register various information necessary to determine the sanitary condition of the work line as the line related information in correspondence with the culture medium information, the data management can be executed such that the culture medium information can be appropriately used for the evaluation of the work line.
(8) Also, in the culture medium information registration system according to the present invention, the culture medium identification information obtaining unit obtains the culture medium information with purpose type information indicating a type of intended use of the culture medium, and the registration unit registers the culture medium information including the obtained purpose type information with the database.
   With this configuration, the culture medium information registration system according to the present invention can execute data management such that information registered according the purpose, such as the information used for analysis, e. g., the normal data analysis or the analysis of the sanitary management of the work line or lot, or the information used when an abnormality in view of the sanitary management occurs, can be used for various data analysis and such that the reliability of the data management can be improved.
(9) In order to solve the above problem, there is provided a program executed by a computer which cooperates with a communication terminal device to register, with a database, culture medium information indicating information of a culture medium on which food produced or inspected by a work line including one or more production process or inspection process is cultured as a specimen, the program making the computer function as: a culture medium identification information obtaining unit which cooperates with the communication terminal device to obtain culture medium identification information for identification from other culture media; a work identification information obtaining unit which obtains work identification information which is inputted by the communication terminal device and which identifies a work of the work line; a search unit which searches line related information related to the work line based on the obtained work identification information, the work related information including information of sanitary management of the work line stored in the database for each work identification information; and a registration unit which registers the culture medium information including at least the obtained culture medium identification information, with the database, in correspondence with the searched line related information.
   With this configuration, since the program according to the present invention can cooperate with the communication terminal device such as a portable communication terminal device to register the culture medium identification information with the database in correspondence with the line related information, the culture medium information and the line related information may be easily registered in correspondence with each other, in comparison with the conventional case where the line related information necessary to produce a report of the culture medium is directly and manually entered by a worker in a predetermined space of a film-type culture medium or a container of the culture medium.
   Therefore, the program according to the present invention can eliminate the complicated work of registering each information to prevent the registration error, and can register the culture medium information in such a manner that the data management becomes easy.
(10) In order to solve the above problem, there is provided a communication terminal device which cooperates with a server system to register, with a database, culture medium information indicating information of a culture medium on which food produced or inspected by a work line including one or more production process or inspection process is cultured as a specimen, the communication terminal device comprising: an image data obtaining unit which obtains image data of the specimen; a culture medium identification information input unit which is used to input culture medium identification information for identifying a culture medium on which the specimen imaged is cultured; a work identification information input unit which is used to input work identification information for identifying a work of the work line; and a communication control unit which executes data communication with the server device and which registers the culture medium information including at least the obtained image data and the culture medium identification information with the database in correspondence with line related information related to the work line and including information of sanitary management of the work line stored in the database for each work identification information .
   With this configuration, since the communication terminal device according to the present invention can cooperate with the server device to register the culture medium identification information with the database in correspondence with the line related information, the culture medium information and the line related information may be easily registered in correspondence with each other, in comparison with the conventional case where the line related information necessary to produce a report of the culture medium is directly and manually entered by a worker in a predetermined space of a film-type culture medium or a container of the culture medium.
   Therefore, the communication terminal device according to the present invention can eliminate the complicated work of registering each information to prevent the registration error, and can register the culture medium information in such a manner that the data management becomes easy.
(11) Also, in the communication terminal device according to the present invention, the image data obtaining unit and the culture medium information input unit are constituted by an imaging unit formed by an imaging element.
   With this configuration, since the communication terminal device according to the present invention can register the image data of the culture medium with the database in correspondence with the culture medium identification information and the line related information, the number of colonies can be detected by the predetermined image analysis of the image data and its information can be registered.
   Therefore, since the communication terminal device according to the present invention can automate the analysis of each culture medium for registration, it is possible to easily register the information enabling not only the evaluation of the specimen cultured in each culture medium but the automatic determination of the sanitary condition of the process, the work line or the lot.
(12) Also, the communication terminal device according to the present invention may further comprise a timer which manages at least a current time, and the image data obtaining unit obtains a time of obtaining the image data as an obtained time information.
   With this configuration, the communication terminal device according to the present invention can use the obtaining time obtaining the image data as the inspection time a predetermined time after the culture start time of the specimen. Therefore, the complicated work of registering each information can be eliminated to prevent the registration error, and the culture medium information can be registered in such a manner that the data management becomes easy.
(13) Also, the communication terminal device according to the present invention may further comprise a process information obtaining unit which obtains process information for specifying at least one of a process of extracting the specimen from the work line and an extraction area of extracting the specimen, and the communication control unit transmits the culture medium information including the obtained process information to the server system.
   With this configuration, the communication terminal device according to the present invention can automatically register the process in which the specimen is extracted or the area from which the specimen is extracted. Therefore, the complicated work of registering each information can be eliminated to prevent the registration error, and the culture medium information can be registered in such a manner that the data management becomes easy.
(14) Also, the communication terminal device according to the present invention may further comprise a detection unit which detects a number of colonies in the specimen included in the image data based on the obtained image data, and the communication control unit transmits the culture medium information including the detected number of colonies to the server system.
   With this configuration, the communication terminal device according to the present invention can detect the number of colonies in the culture medium by analyzing the image data and register the information. Therefore, the analysis of each culture medium can be automated to register the information, and it is possible to register the information enabling, not only the evaluation of the specimen cultured in each culture medium, but also the automatic determination of the sanitary condition of the process, the work line and the lot.
(15) Also, the communication terminal device according to the present invention may further comprise a purpose type information obtaining unit which obtains purpose type information indicating a type of intended use of the culture medium, and the communication control unit transmits the culture medium information including the obtained purpose type information to the server system.
   With this configuration, the communication terminal device according to the present invention can execute data management such that information registered according the purpose, such as the information used for analysis, e.g., the normal data analysis or the analysis of the sanitary management of the work line or lot, or the information used when an abnormality in view of the sanitary management occurs, can be used for various data analysis and such that the reliability of the data management can be improved.
(16) In order to solve the above problem, there is provided a program executed by a computer which cooperates with a server system to register, with a database, culture medium information indicating information of a culture medium on which food produced or inspected by a work line including one or more production process or inspection process is cultured as a specimen, the program making the computer function as : an image data obtaining unit which obtains image data of the specimen; a culture medium identification information input unit which is used to control an input of culture medium identification information for identifying a culture medium on which the imaged specimen is being cultured; a work identification information input unit which is used to control an input of work identification information for identifying a work of the work line; and a communication control unit which executes data communication with the server device and which registers the culture medium information including at least the obtained image data and the culture medium identification information with the database in correspondence with line related information related to the work line and including information of sanitary management of the work line stored in the database for each work identification information.
   With this configuration, since the program according to the present invention can cooperate with the server device to register the culture medium identification information with the database in correspondence with the line related information, the culture medium information and the line related information may be easily registered in correspondence with each other, in comparison with the conventional case where the line related information necessary to produce a report of the culture medium is directly and manually entered by a worker in a predetermined space of a film-type culture medium or a container of the culture medium.
   Therefore, the program according to the present invention can eliminate the complicated work of registering each information to prevent the registration error, and can register the culture medium information in such a manner that the data management becomes easy.
(17) In order to solve the above problem, there is provided a sanitary management system comprising a communication terminal device; and a server device which cooperates with the communication terminal device to register, with a database, culture medium information indicating information of a culture medium on which food produced or inspected by a work line including one or more production process or inspection process is cultured as a specimen, wherein the communication terminal device comprising: an image data obtaining unit which obtains image data of the specimen; a culture medium identification information input unit which is used to input culture medium identification information for identifying a culture medium on which the imaged specimen is cultured; a work identification information input unit which is used to input work identification information for identifying a work of the work line; and a communication control unit which executes data communication with the server device and which registers the culture medium information including at least the obtained image data and the culture medium identification information with the database in correspondence with line related information related to the work line and including information of sanitary management of the work line stored in the database for each work identification information, and wherein the server device comprising: a culture medium identification information obtaining unit which cooperates with the communication terminal device to obtain the culture medium identification information; a work identification information obtaining unit which obtains work identification information inputted by the communication terminal device; a search unit which searches the line related information stored in the database for each work identification information; and a registration unit which registers the culture medium information including at least the obtained culture medium identification information, with the database, in correspondence with the searched line related information.
   With this configuration, since the sanitary management system according to the present invention can register the culture medium identification information with the database in correspondence with the line related information, the culture medium information and the line related information may be easily registered in correspondence with each other, in comparison with the conventional case where the line related information necessary to produce a report of the culture medium is directly and manually entered by a worker in a predetermined space of a film-type culture medium or a container of the culture medium.
   Therefore, the sanitary management system according to the present invention can eliminate the complicated work of registering each information to prevent the registration error, and can register the culture medium information in such a manner that the data management becomes easy.
(18) In order to solve the above problem, there is provided a film-type culture medium comprising: a base material formed by a film; a culture layer which is provided on the base material and on which food produced or inspected by a work line including one or more production process or inspection process is cultured as a specimen; and a mark forming part on which identification information is formed in a manner readable when the identification information is imaged by a communication terminal device, the identification information being unique information used to register and manage information related to at least one of the specimen and a condition of the specimen with the database as culture medium information.

With this configuration, since the film-type culture medium according to the present invention can register the culture medium identification information with the database in correspondence with the line related information, the culture medium information and the line related information may be easily registered in correspondence with each other, in comparison with the conventional case where the line related information necessary to produce a report of the culture medium is directly and manually inputted entered by a worker in a predetermined space of a film-type culture medium or a container of the culture medium.

Therefore, the film-type culture medium according to the present invention can eliminate the complicated work of registering each information to prevent the registration error, and can register the culture medium information in such a manner that the data management becomes easy.

### EFFECT OF THE INVENTION

According to the culture medium information registration system, the communication terminal device, the program therefor and the sanitary management system, complexity of a worker who registers the culture medium information can be eliminated to prevent an registration error of the culture medium information, and the culture medium information can be registered in such a manner that data can be easily managed.

Additionally, the film-type culture medium can be used to eliminate complexity of a worker who registers the culture medium information to prevent a registration error of the culture medium information, and to easily manage data.

### BRIEF DESCRIPTION OF THE DRAWINGS

FIG. 1 is a diagram showing a configuration of an embodiment of a sanitary management system according to the present invention.
FIG. 2 is an example of a film-type culture medium used in the sanitary management system of the embodiment.
FIG. 3 is an example of a work instruction sheet used in the sanitary management system of the embodiment.
FIG. 4 is a block diagram showing a configuration of a portable communication terminal device used in the sanitary management system of the embodiment.
FIG. 5 is an example of an image displayed on a display unit when culture medium information is registered by the portable communication terminal device of the embodiment.
FIGS. 6A and 6B show examples of the image displayed on the display unit when the film-type culture medium is imaged by the portable communication terminal device of the embodiment.
FIGS. 7A and 7B are examples of the image displayed on the display unit when a work instruction sheet is imaged by the portable communication terminal device of the embodiment.
FIG. 8 is a block diagram showing a configuration of a server device used in the sanitary management system of the embodiment.
FIG. 9 is a diagram (part 1) for explaining a process determination in the server device of the embodiment.
FIG. 10 is a diagram (part 2) for explaining the process determination in the server device of the embodiment.
FIG. 11 is a diagram (part 3) for explaining the process determination in the server device of the embodiment.
FIG. 12 is a flowchart showing an operation of registration processing of the culture medium information in the portable communication terminal device of the embodiment.
FIGS. 13A and 13B are examples of the image displayed on the display unit when the culture medium information is transmitted by the portable communication terminal device of the embodiment.
FIG. 14 is a flowchart showing an operation of registration processing of the culture medium information in the server device of the embodiment.
FIG. 15 is a flowchart showing an operation of lot determination processing in the server device of the embodiment.

### DETAILED DESCRIPTION OF THE PREFERRED EMBODIMENTS

A Preferred embodiment of the present invention will be described below with reference to the attached drawings. The following embodiment is directed to a case where a culture medium information registration system, a communication terminal device, programs thereof, a sanitary management system and a film-type culture medium according to the present invention are applied to a sanitary management system using a film-type culture medium in a production line of processed food. However, the present invention is not limited to the following embodiment within a range including its technical idea.

### [1] Sanitary Management System

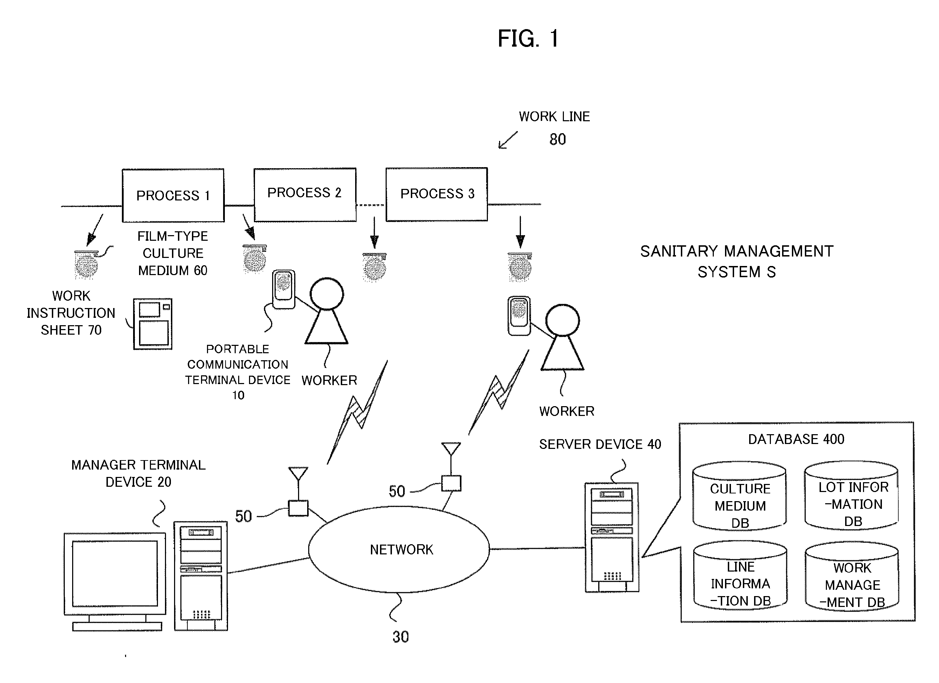

First, a sanitary management system S according to the embodiment will be described with reference to FIG. 1. FIG. is a block diagram illustrating a configuration of the sanitary management system S of the embodiment.

As shown in FIG. 1, the sanitary management system of the embodiment includes a plurality of portable communication terminal devices 10 used for registering a film-type culture medium 60, a manager terminal device 20 which manages the sanitary management system S, a network 30, and a server device 40 which cooperates with the portable communication terminal devices 10 or the manager terminal device 20 to execute various processing.

The sanitary management system S is configured to extract food produced or inspected by using a work line 80 including one or more production process or inspection process as a specimen, and register information of the culture medium (hereinafter referred to as "culture medium information") obtained by the portable communication terminal device 10 before and after culturing the extracted specimen with the server device 40 (specifically, a database 400) in correspondence with information (hereinafter referred to as "work line related information") related to the work line 80 including information of the sanitary management of the work line 80 from which the specimen is extracted.

This work line related information includes line information for specifying the work line 80 from which the specimen is extracted, process information for specifying the process that extracted the specimen, and lot information for specifying the lot including the specimen. Also, the work line related information includes, as the information of the sanitarymanagement of the work line 80, a specimen type indicating a type of the specimen, a culture start time of the specimen, a dilution rate at the time of culturing the specimen in the culture medium, and a determination of normality or abnormality of the specimen in each film-type culture medium 60 (hereinafter referred to as "culture medium determination"), i.e., information on a criterion used at the time of determining acceptance/rejection (hereinafter referred to as "culture medium determination criterion information").

In the sanitary management system S of the embodiment, the server device 40 cooperates with the portable communication terminal device 10 to obtain culture medium identification information 65 (hereinafter referred to as "culture medium ID") applied to each culture medium for identifying the culture medium from others and obtain work identification information (hereinafter referred to as "work ID") inputted by a worker for identifying the work in the work line 80. Then, the server device 40 searches the line related information stored in the database 400 for each of the work IDs 71, and registers the culture medium information including the obtained culture medium ID 65 with the database 400 in correspondence with the searched line related information.

Also, the server device 40 uses instruction sheet identification information (hereinafter referred to as "instruction sheet ID") 71 applied to each of work instruction sheets 70 or identification information (hereinafter referred to as "process ID") 72 of process specifying each process in each work line 80, as the work ID 71, and searches the line related information based on the obtained work ID 71 to register the culture medium information with the database 400 in correspondence with the line related information specified by the search.

In this embodiment, identification information for specifying the work line 80 (hereinafter referred to as "line ID") may be used as the work ID 71. Also, plural IDs of the instruction sheet ID 71 and the process ID 72 may be used as the work ID 71.

On the other hand, the sanitary management system S can execute the determination of sanitary condition of each process of the work line 80 (hereinafter referred to as "process determination"), the determination of sanitary condition of the work line 80 (hereinafter referred to as "line determination") or the determination whether or not the food group produced or inspected under the same condition (i.e., the food group belonging to one lot") satisfies a predetermined condition (hereinafter referred to as "lot determination").

Particularly, in the sanitary management system S of this embodiment, the server device 40 obtains image data of each of plural specimens imaged together with the culture medium ID 65, obtains the work ID 71, and detects a number of colonies (hereinafter referred to as "a number of colonies") in the specimen based on the obtained image data.

Also, the server device 40 searches the line related information of the work line 80, from which the specimen imaged into each image data is extracted, from the database 400 based on the obtained work ID 71.

Then, the server device 40 determines whether or not the food group (i.e., the lot) produced or inspected under the same condition satisfies the predetermined condition, based on the number of colonies in the specimen of the detected image data and the searched line related information, and provides the determination result.

The culture medium information is the information that is transmitted to the server device 40 by the portable communication terminal device 10 and is registered with the database 400. Specifically, the culture medium information includes the culture medium ID 65, the image data (hereinafter referred to as "culture medium image data") obtained by imaging the specimen before the culture in the film-type culture medium 60 or the specimen after a predetermined time has passed from the culture, metadata such as the imaging time and the image data ID, the number of colonies in the cultured specimen, and information indicating the result of the culture medium determination (hereinafter referred to as "culture medium determination information") indicating normality or abnormality of the specimen in view of the sanitary management.

Also, the lot information includes the instruction sheet ID 71 (hereinafter referred to as "lot ID") unique to the work and identifying the lot from other lots, the line information used to produce or inspect the food belonging to each lot, the time of starting the production or inspection of the lot (hereinafter referred to as "lot start time"), the time of ending the production or inspection of the lot (hereinafter referred to as "lot end time"), and a party of workers engaged in the production or inspection of the lot (hereinafter referred to as "work party").

Further, the line information includes the line ID specifying other work lines 80, and processing type specifying each process and its process ID 72.

The portable communication terminal device 10 is a communication terminal device such as a digital camera having a communication function, a tablet-type information terminal device, a smartphone or a mobile phone, which has an imaging or recording function of still images (hereinafter referred to as "camera function") and is portable by the worker.

Particularly, the portable communication terminal device 10 can image the film-type culture medium 60, image the culture medium ID 65 together with the film-type culture medium 60 and image the work instruction sheet 70 including the work ID 71, by its camera function.

Also,the portable communication terminal device 10 obtains the culture medium ID 65 and the work ID 71 from the culture medium image data and the instruction sheet image data, and transmits the culture medium image data of the film-type culture medium 60 and the metadata of the culture medium image data to the server device 40 as the culture medium information, together with the obtained culture medium ID 65 and the obtained work ID 71.

Particularly, the portable communication terminal device 10 transmits the culture medium information to the server device 40 directly or via access points 50 by using a short-range wireless communication standard such as BLUETOOTH (Registered Trademark), wireless LAN (WLAN: Wireless Local Area Network) or wireless PAN (WPAN: Wireless Personal Area Network), or by using a public telephone network via a mobile base station not shown.

Also, the portable communication terminal device 10 has a browser function configured by the markup language such as XML (eXtensible Markup Language), executes operation input instruction and operation confirmation of the worker by using the browser function, and transmits the culture medium information to the server device 40 via the browser function.

In a case where each of the film-type culture medium 60 and the work instruction sheet 70 has an IC tag and each IC tag stores the culture medium ID 65 and the work ID 71, the portable communication terminal device 10 may read out and obtain the culture medium ID 65 and the work ID 71 from the ID tag and transmit them to the server device 40 together with the culture medium image data.

Also, the portable communication terminal device 10 may transmit the culture medium image data of the imaged film-type culture medium 60 including the culture medium ID 65 and the instruction sheet image data of the imaged work instruction sheet 70 to the server device 40 in a predetermined data format, and the server device 40 may analyze and obtain the work ID 71 and the culture medium ID 65.

The manager terminal device 20 is an information communication terminal device such as a tablet-type information terminal device, asmartphone, a personal computer or a workstation. Particularly, the manager terminal device 20 has a monitor and/or a printer to output an analysis result when a report related to the sanitary management of the lot and the work line 80 is produced or when other data analyses are executed.

The manager terminal device 20 manages the ID and password of the manager, and executes management of the access authorization to the server device 40, management of the terminal IDs of the portable communication terminal devices 10, correction of the registered culture medium information and other management. The manager terminal device 20 has the browser function configured by the markup language similarly to the portable communication terminal device 10, and can execute data communication with the server device 40 and browsing of the reports by using the browser function.

The manager terminal device 20 can correct the culture medium information and other information when each work is terminated due to a trouble of a machine performing the process or other reasons in the same lot or when the registration error of the culture medium information is made by the worker.

For example, the network 30 is configured by a public telephone network (hereinafter referred to as "a long distance communication network") including a mobile communication network, an IP (Internet Protocol) network such as a short-range wireless network, or both of them connected to each other. However, the configuration of the network 30 is not limited to them.

The server device 40 cooperates with the manager terminal device 20 to execute the sanitary management of the lot and the work line 80, and cooperates with the portable communication terminal device 10 to execute the data management of the culture medium information.

Particularly, the server device 40 receives the culture medium image data generated by imaging the film-type culture medium 60 and its metadata from the portable communication terminal device 10 together with the culture medium ID 65 and the work ID 71, and specifies the line related information (i.e., the lot information, the line information and the culture medium information) stored in correspondence with the work ID 71.

The server device 40 obtains the imaging time based on the metadata of the received culture medium image data, obtains or detects the number of colonies in the imaged specimen, and registers each of the culture medium information such as the culture medium ID 65, the imaging time and the number of colonies, in correspondence with the line related information searched by the work ID 71.

The server device 40 may receive the culture medium image data by imaging the film-type culture medium 60 and the instruction sheet image data generated by imaging the work instruction sheet 70 by the portable communication terminal device 10, and analyze the culture medium image data and the instruction sheet image data to recognize and obtain the work ID 71 and the culture medium ID 65.

On the other hand, the server device 40 can cooperate with the manager terminal device 20 to execute data analysis related to the sanitary management in the designated lot, work line 80 or process, and output analysis result in the report format (hereinafter referred to as "analysis information").

Specifically, the server device 40 determines abnormality or normality of the lot, the work line 80 or each process included in the work line 80 (hereinafter simply referred to as "process") in view of the sanitary management (i.e., acceptance/rejection of the lot or the work line 80 in view of the sanitary management), based on the plural culture medium information registered in correspondence with the lot, the work line 80 or the process to be analyzed, and generates the analysis result in the report format based on the determination result.

Then, the server 40 can transmit the generated analysis result to the manager terminal device 20 in such a manner that the manager can browse.

Also, the server device 40 executes each determination by using culture medium determination criterion information used as the criterion at the time of executing the culture medium determination, line determination criterion information used as the criterion at the time of executing the line determination, process determination criterion information used as the criterion at the time of executing the process determination, and lot determination criterion information used as the criterion at the time of executing the lot determination.

The determination criterion information of each kind is the criterion information for executing the determination prescribed by the number of lots, the number of work lines, the process type, the specimen type, the kind of bacterium detected from the specimen, the dilution rate at the time of culture, and the culture time (an elapsed time from the start of the culture), at the time of executing the culture medium determination.

Then, the server device 40 reads out the determination criterion information different between the number of lot, the number of work lines, the process type, the specimen type, the kind of bacterium detected from the specimen, the dilution rate at the time of culture, and the culture time (the elapsed time from the start of the culture), to execute each of the culture medium determination, the lot determination, the line determination and the process determination.

With the above configuration, the sanitary management system S of this embodiment can cooperate with the portable communication terminal device 10 to register the culture medium ID 65 with the database 400 in correspondence with the line related information. Therefore, it is possible to easily associate and register the culture medium information compared with the conventional case, in which the worker directly enters the work line 80 and other various information necessary to produce a report of the culture medium in a predetermined space of the film-type culture medium 60 or on the container of the film-type culture medium.

Therefore, the sanitary management system S of this embodiment can eliminate the complicated work of registering each information to prevent errors in registering the culture medium information, and can register the culture medium information in such a manner that the data management can be easily performed.

### Film-type Culture Medium

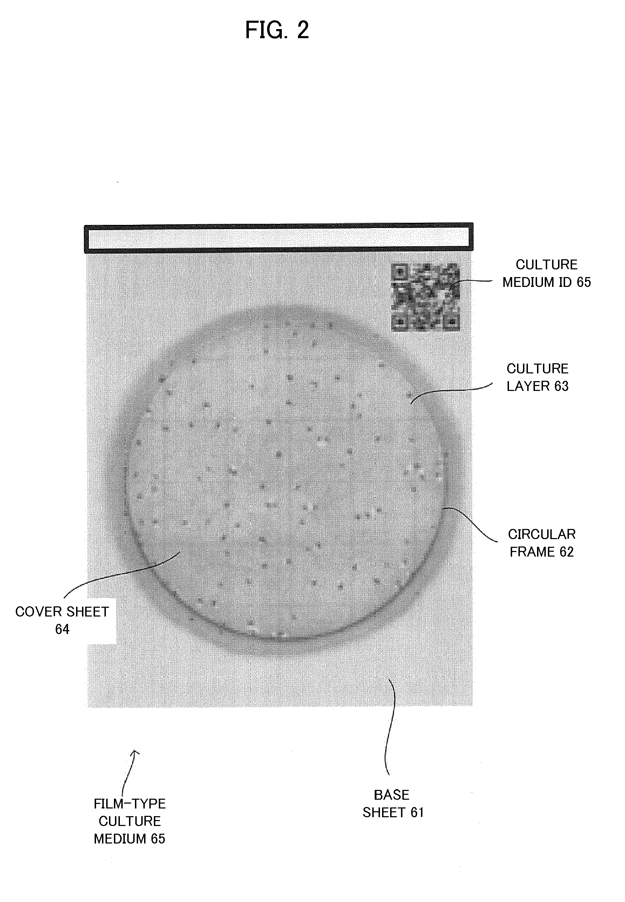

Next, the film-type culture medium 60 of this embodiment will be described with reference to FIG. 2. FIG. 2 is an example of the film-type culture medium 60 used in this embodiment.

The film-type culture medium 60 used in this embodiment is a culture medium to culture food detected from each process of the work line 80 by a dried culture medium like a film or a sheet as a specimen. The film-type culture medium 60 is used as a culture medium to culture viable microorganisms, coliform bacteria and Staphylococcus aureus. The film-type culture medium 60 may be used as a culture medium to culture various kinds of bacteria, substance or microorganism such as mold, yeast, Listeria monocytogenes, water microorganism, lactic acid bacteria, and protein.

As shown in FIG. 2, the film-type culture medium 60 includes a base sheet 61 formed of a film, a circular frame (hereinafter referred to as "circular frame") 62 formed on the base sheet 61 from the center of the base sheet 61, a culture layer 63 provided inside the frame, and a cover sheet 64 to cover the culture layer 63.

The film-type culture medium 60 of this embodiment includes a culture medium ID 65 formed at the upper part of the base sheet 61 by printing or other method and serving as identification information of the film-type culture medium 60. Particularly, the culture medium ID 65 functions as a mark forming part formed on the base sheet 61, which is readable when the culture medium 60 is imaged by the portable communication terminal device 10. For example, the culture medium ID 65 is formed on the upper part of the base sheet 61 as a barcode such as a two-dimensional barcode or alphanumeric characters. The culture medium ID 65 is imaged when the film-type culture medium 60 is imaged, and is recognizable by the analysis of the portable communication terminal device 10 or the server device 40.

The base sheet 61 is a base material of a film or a sheet, and its material is not limited. For example, a plastic film or a paper may be used. Preferably, an example of the plastic film is a resin film such as polyethylene, polypropylene, polyethylene-telephthalate, polyethylene-naphthalate, polymethacrylate, polymethyl methacrylate, polymethyl acrylate, polyester, and polycarbonate. Polyethylene-telephthalate or a synthetic paper of polypropyleneispreferred. The synthetic paper of polypropylene is a film synthetic paper mainly made from polypropylene.

The film-type culture medium 60 may include an IC tag storing the culture medium ID 65, and the portable communication terminal device 10 may obtain the culture medium ID 65 by a short-range wireless communication interface 140 (e.g., an IC tag reader) provided in the portable communication terminal device 10.

### [3] Work Instruction Sheet

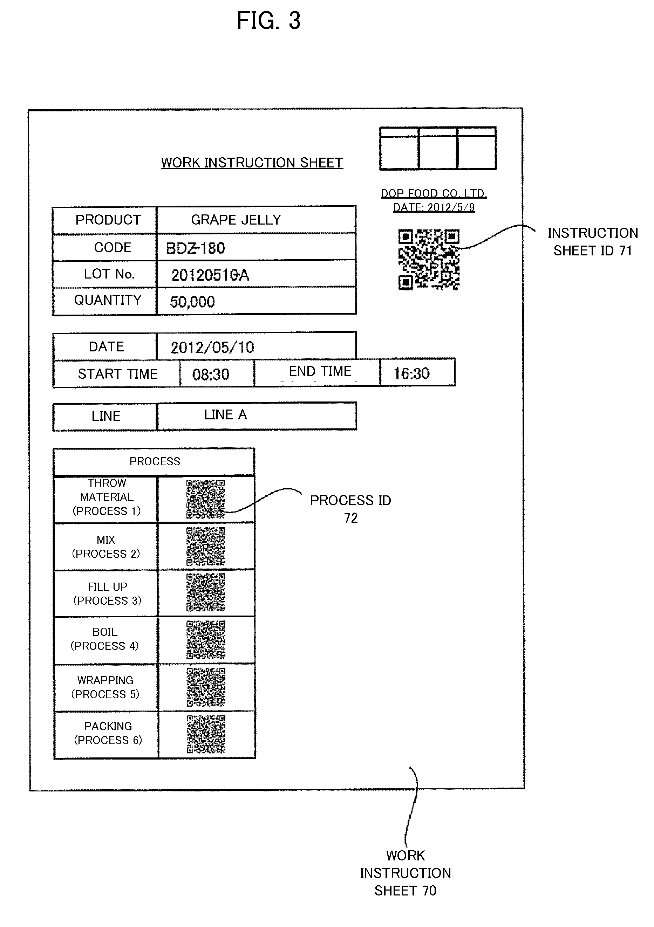

Next, the work instruction sheet 70 of this embodiment will be described with reference to FIG. 3. FIG. 3 is an example of the work instruction sheet 70 used in this embodiment.

The work instruction sheet 70 of this embodiment is an instruction sheet on which work names and contents of one or more production process or inspection process, the work line 80 used, a name of food (processed food) produced or inspected, a quantity and other information are written for each group of food produced or inspected under the same condition, i.e., for each lot.

The work instruction sheet 70 of this embodiment includes an instruction sheet ID 71 formed by printing or other method at the upper part, and plural process IDs 72 formed by printing or other method at predetermined areas. The instruction sheet ID 71 and each of the process IDs 72 are used as the work ID 71, and is formed by a barcode such as a two-dimensional barcode or alphanumeric characters, for example. The instruction sheet ID 71 and each of the process IDs 72 are imaged when the film-type culture medium 60 is registered, similarly to the culture medium 60, and are recognized by the analysis in the portable communication terminal device 10 or the server device 40.

Similarly to the film-type culture medium 60, the work instruction sheet 71 may include an IC tag storing the work ID which is the instruction sheet ID 71 or the process ID 72, and the portable communication terminal device 10 may obtain the work ID 71 by an interface (a short-range wireless communication interface 140 described later) such as a tag reader provided in the portable communication terminal device 10.

### [4] Portable Communication Terminal Device

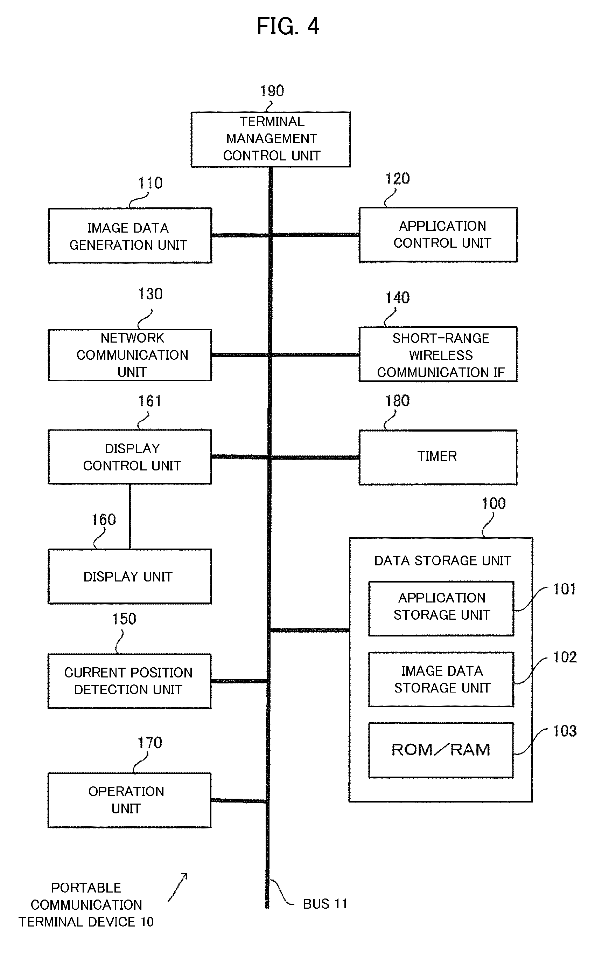

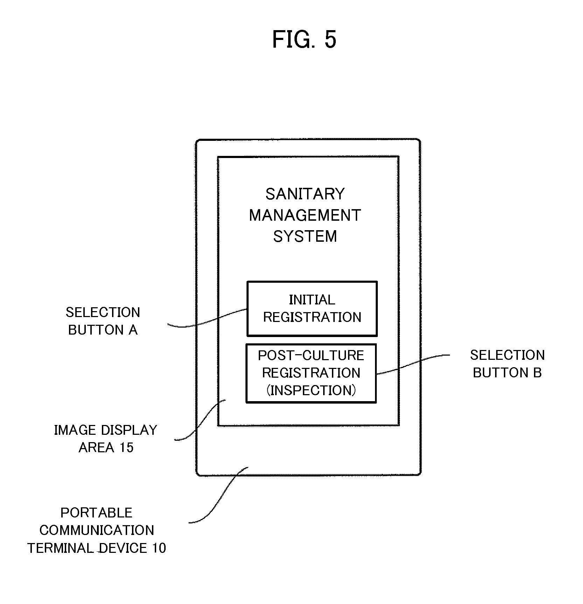

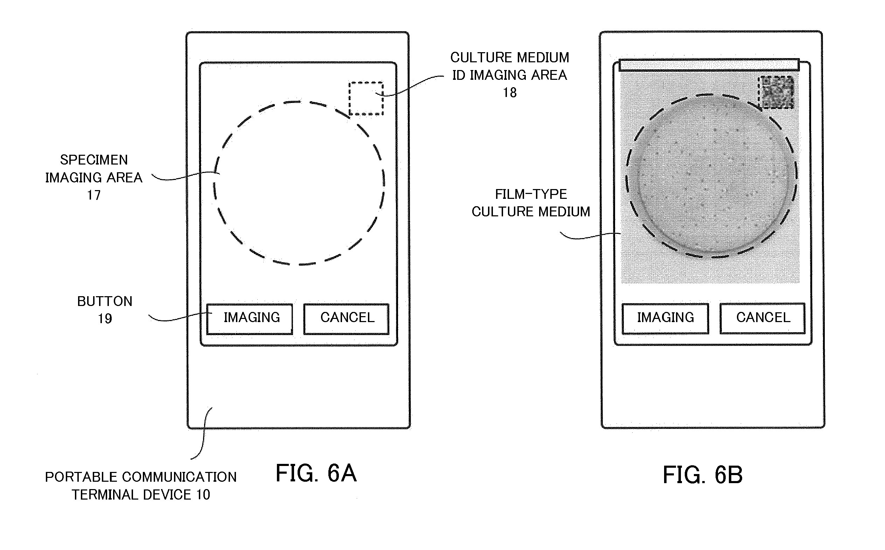

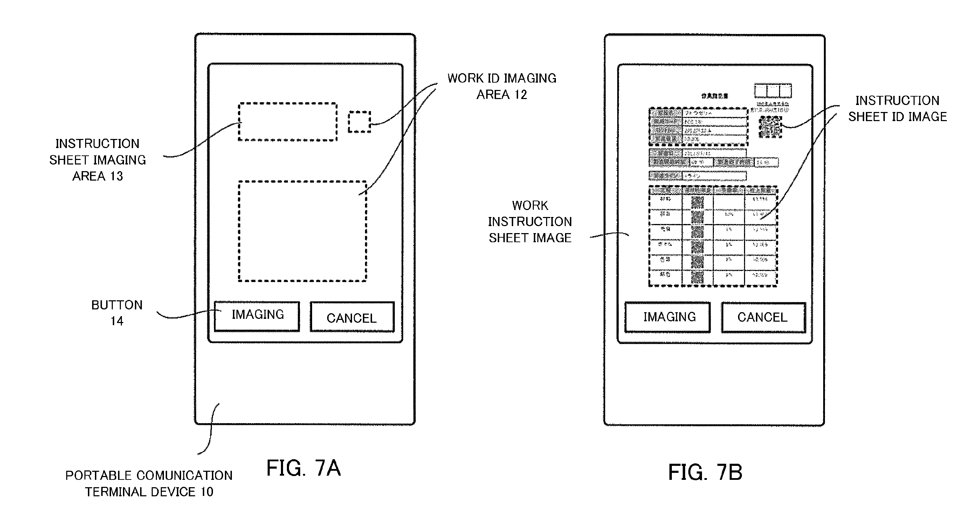

Next, the configuration of the portable communication terminal device 10 of this embodiment will be described with reference to FIGS. 4 to 7. FIG. 4 shows the configuration of the portable communication terminal device 10 of this embodiment, and FIG. 5 is an example of an image displayed on a display unit 160 when the portable communication terminal device 10 of this embodiment registers the culture medium information. FIGS. 6A and 6B are examples of images displayed on the display unit 160 when the portable communication terminal device 10 of this embodiment images the film-type culture medium 60, and FIGS. 7A and 7B are examples of images displayed on the display unit 160 when the portable communication terminal device 10 of this embodiment images the work instruction sheet 70.

The portable communication terminal device 10 of this embodiment includes a data storage unit 100 which has a memory function used when various programs are executed, an image data generation unit 110 which has an imaging function and which generates the image data of the film-type culture medium 60 and other image data, and an application control unit 120 which cooperates with the server device 40 to execute the registration processing of the culture information.

Also, the portable communication terminal device 10 includes a network communication unit 130 which communicates with the server device 40 and other communication device, a short-range wireless communication interface 140 which transmits and receives data to and from the IC tag and other communication interface, a current position detection unit 150 which detects the current position, a display unit 160, a display control unit 161 which controls the display unit 160, an operation unit 170 for inputting user's operation, a timer 180, and a terminal management control unit 190 which controls the whole device.

For example, if the portable communication terminal device 10 has a telephone function and a mailing function such as an e-mail, it includes various necessary elements such as a microphone, a speaker and an e-mail transmitting/receiving function. Further, each of the above-described units is connected to each other by a bus 11 to transmit and receive data.

For example, the image data generation unit 110 constitutes an image data obtaining unit, a culture medium identification information input unit, a work information input unit and an imaging unit according to the present invention. The application control unit 120 constitutes a communication control unit, a culture medium identification information input control unit and a work information input control unit according to the present invention. For example, the short-range wireless communication interface 140 constitutes the image data obtaining unit, the culture medium identification information input unit and the work information input unit according to the present invention.

The data storage unit 100 includes an application storage unit 101 which stores various application programs, an image data storage unit 102 which stores image data imaged and generated by the image data generation unit 110, and ROM/RAM 103 which stores programs related to the management and control of the portable communication terminal device 10 and which is used as a work area during the execution of each program.

Particularly, the application storage unit 101 stores application programs (hereinafter referred to "App") executed by the application control unit 120 in cooperation with the image data generation unit 110, the operation unit 170, the display control unit 161 and the image data storage unit 102. Also, the application storage unit 101 stores browser program to realize the browsing function described above.

The image data storage unit 102 stores the culture medium image data, the image ID for managing the image data, various metadata such as the imaging time corresponding to each image data, and the instruction sheet image data, in a manner associated with each other. The image ID is arbitrary identification information suitably given in each of the portable communication terminal devices 10.

The image data generation unit 110 includes an optical system, a CCDI (Charge Coupled Device Image) sensor which converts an optical image inputted from the optical system to an electric signal, and a generation unit which generates the image data based on the electric signal generated by the CCDI sensor.

Particularly, when the film-type culture medium 60 on which the specimens are cultured or being cultured is imaged together with the culture medium ID 65, the image data generation unit 110 images the the film-type culture medium 60 to generate the culture medium image data of the film-type culture medium. In addition, when the work instruction sheet 70 is imaged, the image data generation unit 110 images the work instruction sheet 70 to generate the instruction sheet image data of the work instruction sheet 70.

The application control unit 120 realizes culture medium information registration processing executed by a culture information registration processing program (hereinafter referred to as "culture medium registration application"). Particularly, the application control unit 120 executes various control programs for controlling each unit of the portable communication terminal device 10 by the culture medium registration application stored in the application storage unit 101, and cooperates with or controls the network communication unit 130, the display control unit 161 and the operation unit 170 to execute various processing.

Specifically, the application control unit 120 executes the culture medium registration application stored in the application storage unit 101, controls the image data generation unit 110 to obtain the culture medium image data of the film-type culture medium 60 and the instruction sheet image data of the work instruction sheet 70, and stores them in the image data storage unit 102.

Then, the application control unit 120 obtains the culture medium ID 65 and the work ID 71 from the culture medium image data and the instruction sheet image data by using an image analyzing function such as a barcode recognition function or an OCR (Optical Character Recognition) function, and stores the culture medium image data of the film-type culture medium 60 and the metadata of the culture medium image data into the image data storage unit 102 together with the culture medium ID 65 and the work ID 71 thus obtained.

The application control unit 120 may execute the barcode recognition or the OCR recognition in cooperation with another communication device or a database via a communication line.

Also, after imaging the culture medium image data or at a predetermined timing, the application control unit 120 executes initial registration which registers the culture medium information to the server device 40 at the time of starting the culture of specimen and post-culture registration which registers the culture medium information after starting the culture of the specimen. Particularly, the application control unit 120 specifies the initial registration or the post-culture registration based on the instruction by the worker, and transmits the information (flag information) indicating one of the initial registration and the post-culture registration to the server device 40 in a manner included in the culture medium information.

Also, after imaging the culture medium image data, the application control unit 120 obtains the current time from the timer 180, stores the current time in the image data storage unit 102 as the imaging time together with a predetermined image ID as the metadata of the culture medium image data, and transmits it to the server device 40 together with the culture medium image data transmitted to the server device 40.

For example, as shown in FIG. 5, the application control unit 120 makes the worker who registers the culture medium information select one of the initial registration and the post-culture registration, by detecting the touch on the display of the selection buttons A and B and the corresponding area of the image display area 15. When the culture medium ID 65 is formed by a two-dimensional barcode, the application control unit 120 displays the area for imaging the specimen (hereinafter referred to as "the specimen imaging area") 17 at the center of the display unit 160 and the area for imaging the culture medium ID 65 (hereinafter referred to as "the culture medium ID imaging area") 18, as shown in FIG. 6A and 6B, so as to invite the worker to image. Then, the application control unit 120 makes the image data generation unit 110 image the culture medium image having the culture medium ID 65 based on the instruction of the worker (i.e., upon detecting the touch on the imaging button 19).

When the application control unit 120 obtains the data of the work instruction sheet 70 on which the instruction sheet ID 71 or the process ID 72 is formed by the two-dimensional barcode as the work ID 71, the application control unit 120 displays the area for imaging the instruction sheet ID 71 or the process ID 72 (hereinafter referred to as "the work ID imaging area") 12 and the area for imaging the work instruction sheet 70 (hereinafter referred to as "instruction sheet imaging area") 13 on the display unit 160, as shown in FIGS. 7A and 7B, so as to invite the worker to image. Then, the application control unit 120 makes the image data generation unit 110 image the work instruction sheet 70 having the work ID 71 based on the instruction of the worker (i.e., upon detecting the touch on the imaging button).

At this time, when the process ID 72 is obtained as the work ID 71 and plural process IDs 72 are formed, the application control unit 120 makes the worker select one of the process IDs 72 and obtains the corresponding process ID 72. However, the application control unit 120 may cooperate with the server device 40 to make the worker refer to the line related information and obtain the process ID to be obtained based on the instruction of the worker.

When the culture medium ID 65 and the work ID 71, which is the instruction sheet ID 71 or the process ID 72, are stored in the IC tag, the application control unit 120 cooperates with the operation unit 170, the display control unit 161 and the short-range wireless communication interface 140 to obtain the culture medium ID 65 and the work ID 71.

Also, when the server device 40 recognizes the culture medium ID 65 and the work ID 71 from the culture medium image data and the instruction sheet image data, the application control unit 120 transmits the instruction sheet image data as the culture medium information in addition to the culture medium image data.

Further, the application control unit 120 may separately transmit the culture medium information and the work ID 71 to the server device 40. In this case, the application control unit 120 transmits the culture medium information and the work ID 71 every time it obtains them.

The network communication unit 130 configures the communication line with the server device 40 connected to the network 30 under the control of the application control unit 120 and the terminal management control unit 190, and transmits and receives various data such as the culture medium image data and the instruction sheet image data.

The short-range wireless communication interface 140 executes the short-range wireless communication by the IC tag under the control of the application control unit 120 and the terminal management control unit 190.

The current position detection unit 150 recognizes the positions of the GPS (Global Positioning System) satellites via the network 30 under the control of the application control unit 120 and the terminal management control unit 190, and detects the satellite signals (GPS signals) transmitted from the GPS satellites.

Then, the current position detection unit 150 calculates (i.e., detects) coordinate values indicated by the latitude and longitude of the current position P of the portable communication terminal device 10 based on the detected GPS signals. Also, the current position detection unit 150 supplies the calculated coordinate values to the application control unit 120 as the position information.

When the portable communication terminal device 10 has a telephone function or a short-range wireless communication function, the current position of the portable communication terminal device 10 may be calculated (detected) based on the direction and the intensity of the radio wave for the telephone or the short-range wireless communication received by a telephone base station.

The display unit 160, which has an image display area of a predetermined size (e.g., 5 inches, W480×H960 pixels) and is constituted by a panel of a liquid crystal or EL (Electro Luminescence), displays predetermined images based on the display data generated by the display control unit 161. Particularly, in this embodiment, the display unit 160 cooperates with the operation unit 170 to display various information and the image of the film-type culture medium 60 while the culture medium registration application is being executed.

The display control unit 161 generates drawing data necessary to draw a predetermined image on the display device 160 and outputs the generated drawing data to the display unit 160 under the control of the application control unit 120 and the terminal management control unit 190.

The operation unit 170 includes various confirmation buttons, operation buttons for inputting each operation instruction, plural keys such as ten-key and a touch sensor provided on the display unit 160, and is used when each operation is performed. Specifically, the operation unit 170 is used to perform the operation for executing various processing described above at the time of activating the culture medium registration application.

In this embodiment, the operation unit 170 may be used when the culture medium ID 65 and the work ID 71 are directly inputted by hand.

The timer 180 provides the application control unit 120 with the date and time at which the image data generation unit 110 images the film-type culture medium 60.

The terminal management control unit 190 is mainly constituted by a central processing unit (CPU) and includes various input/output ports such as a key input port and a display control port. The terminal management control unit 190 totally controls the general function of the portable communication terminal device 10 and the general function to execute the information providing program.

### [5] Server Device

### Configuration of Server Device

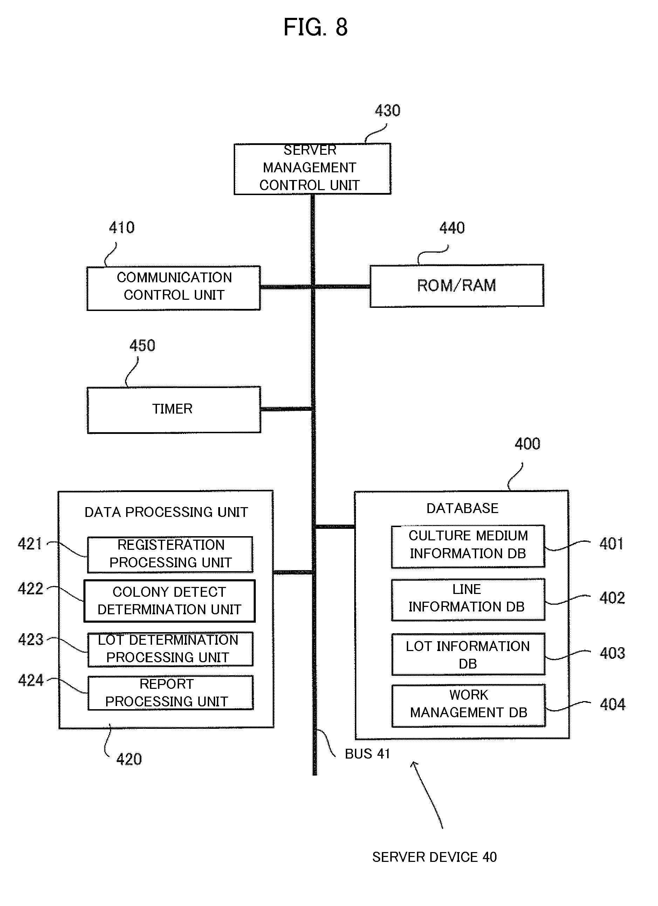

Next, a configuration of the server device 40 of this embodiment will be described with reference to FIG. 8. FIG. 8 is a diagram showing a configuration of the server device 40 of this embodiment.

As shown in FIG. 8, the server device 40 of this embodiment includes a database 400 including a culture medium information database (hereinafter referred to as "the culture medium DB") 401, a line information database (hereinafter referred to as "the line information DB") 402, a lot information database (hereinafter referred to as "the lot information DB") 403 and a work management database (hereinafter referred to "the work management DB") 404; a communication control unit 410 which communicates with the portable communication terminal device 10; a data processing unit 420 which executes various processing such as the culture medium information registration processing and the lot determination processing; a server management control unit 430 which controls each unit of the server device 40; ROM/RAM 440 used for the control of each unit; and a timer 450 used for the time management.

Each unit described above is mutually connected by a bus 41 to execute data transfer between each constitutive element.

The communication control unit 410 is a predetermined network interface, which configures a communication network with the portable communication terminal unit 10 and transmits and receives various data to and from the portable communication terminal device 10.

The database 400 is formed by a HDD, in which the culture medium information DB 401, the line information DB 402, the lot information DB 403 and the work management DB 404 are configured.

The culture medium information DB 401 stores the culture medium information related to the film-type culture medium 60 of the specimen extracted in each process of each work line 80, for each line ID and culture medium ID 65. For example, the culture medium information DB 401 stores the following data in correspondence with each other.
(1) Culture medium ID
(2) Culture medium image data imaged (or re-drawn)
(3) Imaging time
(4) Other information including flag information
(5) A number of colonies
(6) Culture medium determination result
(7) Work ID (Instruction sheet ID or Process ID)

In a case where the specimen to be extracted is constantly extracted from the same process in the same work line 80 in the same lot, the work ID 71 may be the instruction sheet ID 71. In a case where the specimen to be extracted is extracted from the different work line 80 or the different process in the same lot, the work ID 71 is the process ID 72.

Also, the number of colonies and the culture medium determination result may be registered at the time when the culture medium information is registered, or may be registered at the predetermined timing different from the timing of registering the culture medium information.

Further, in a case where plural imaging time are registered for the culture medium information of the same film-type culture medium 60 (i. e., in a case where there are plural culture inspection time described later), each culture medium information including the culture medium image, the imaging time, the number of colonies and the culture medium determination result is stored for each culture medium inspection time in the same culture medium ID 65.

The line information DB 402 stores the line information related to each work line 80 for each line ID. For example, the line information DB 402 stores the following data in correspondence with each other.
(1) Line ID
(2) Process ID of the process in the work line and its type.
As the process type, the name specifying each process including material charging, mixing, boiling, wrapping and packing is used.

The lot information DB 403 stores the lot information related to each lot for each instruction sheet ID 71. For example, the lot information DB 403 stores the following data in correspondence with each other.
(1) Instruction sheet ID (i.e., lot ID)
(2) Line ID of the work line used for production or inspection of the lot
(3) Work start time of the lot
(4) Work end time of the lot
(5) Work party of the workers who perform the work of the lot

The work management DB 404 stores various information related to the work contents specified by each work instruction sheet 70 for each instruction sheet ID 71. For example, the work management DB 404 stores the following data in correspondence with each other.
(1) Instruction sheet ID
(2) Line ID and Process ID of the work line from which specimen is to be extracted
(3) Type of specimen (food)
(4) Dilution rate
(5) Culture start time
(6) Time when a predetermined time has passed after starting the culture (hereinafter referred to as "the culture inspection time")

As the culture inspection time, a single time (i.e., an ending time for ending the culture of the specimen) may be set, or plural time after passing a statutory or predetermined time from the culture start time, e.g., 24 hours after the culture start time or 48 hours after the culture start time, may be set. The lot ID and the line ID are read out as the work list for registering the culture information when the culture medium information is registered, and are used for confirmation to the workers.

The data processing unit 420 executes various data processing in accordance with the application stored in the ROM/RAM 440. Particularly, the data processing unit 420 executes a predetermined program to execute the operation management of the communication control unit 410, the registration processing which registers the culture information including the image data obtained by the portal communication terminal device 10 with the culture information DB 401 in cooperation with each portable communication terminal device 10, the colony detection determination processing which detects the number of colonies in each culture medium image data, the lot determination processing which determines the lot based on the registered culture medium information, the report processing which outputs the determination result in a form of a report, and the management and control of the database 400. Specifically, the data processing unit 420 includes a registration processing unit 421, a colony detection determination unit 422, a lot determination processing unit 423 and a report processing unit 424.

For example, the registration processing unit 421 of this embodiment constitutes a culture medium identification information obtaining unit, a work identification information obtaining unit, a search unit, a registration unit, an image data obtaining unit, a time information obtaining unit and a process information obtaining unit. For example, the colony detection determination unit 422 of this embodiment constitutes a detection unit. The registration processing unit 421, the colony detection determination unit 422, the lot determination processing unit 423 and the report processing unit 424 of this embodiment will be described later in more detail.

The server management control unit 430 is mainly constituted by a central processing unit (CPU), and executes programs for an integrated control of each unit in the server device 40. Specifically, the server management control unit 430 executes login processing of each user based on the login request from the portable communication terminal device 10 by the user's operation, and performs other various controls.

The ROM/RAM 440 stores various programs necessary for the operation of the server device 40. Particularly, the ROM/RAM 440 stores the determination criterion information including the culture medium determination criterion information, the process determination criterion information, the line determination criterion information and the lot determination criterion information. Also, the ROM/RAM 440 is used as a work area during the execution of each program.

The timer 450 is used to manage necessary time when the registration processing of the culture medium information and the lot determination information are executed.

### Registration processing unit

Next, the detail of the registration processing unit 421 in the server device 40 of this embodiment will be described.

The registration processing unit 421 cooperates with the portable communication terminal device 10 to execute the registration processing for registering the culture medium information including the culture medium image data transmitted with the work ID, with the culture medium information DB 401, based on the work ID transmitted from the portable communication terminal device 10 via the communication control unit 410.

Particularly, the registration processing unit 421 executes different culture medium information registration processing based on the flag information included in the culture medium information and indicating one of the initial registration and the post-culture registration.

### (Initial Registration)

When the flag information indicating the initial registration is included in the culture medium information transmitted from the portable communication terminal device 10, the registration processing unit 421 executes the registration processing based on the initial registration.

Specifically, when the registration processing unit 421 obtains the work ID 71 and the culture medium information via the communication control unit 410, the registration processing unit 421 searches the line information DB 402, the lot information DB 403 and the work management DB 404 based on the obtained work ID 71, specifies the line related information of the work line 80 from which the specimen is extracted, and registers the culture medium ID 65, the culture medium image data and the imaging time included in the culture medium information with the culture medium information DB 401 in correspondence with the specified line related information.

Particularly, the registration processing unit 421 specifies the lot ID including the work line 80 from which the specimen is extracted, the line ID of the work line 80 and the process ID 72 in which the specimen is extracted, based on the obtained work ID 71, and registers the culture medium ID 65, the culture medium image data and the imaging time included in the culture medium information with the culture culture medium information DB 401 in correspondence with the lot ID, the line ID and the process ID 72 thus specified.

Then, the registration processing unit 421 registers the imaging time with the culture medium information DB 401 as the culture start time in correspondence with the specified line ID. On the premise that the registration processing of the culture medium information is executed at the time, a preset time may be used as the culture start time, instead of the imaging time.

### (Post-culture registration)

When the flag information indicating the post-culture registration is included in the culture medium information transmitted from the portable communication terminal device 10, the registration processing unit 421 executes the registration processing based on the post-culture registration.

Specifically, the registration processing unit 421 obtains the work ID 71 and the culture medium information via the communication control unit 410 similarly to the initial registration, and searches the line information DB 402, the lot information DB 403 and the work management DB 404 based on the obtained work ID 71. Then, the registration processing unit 421 specifies the line related information of the work line 80 from which the specimen is extracted, and registers the culture medium ID 65, the culture medium image data and the imaging time included in the culture medium information with the culture medium information DB 401 in correspondence with the specified line related information.

Also, the registration processing unit 421 registers the imaging time with the culture medium information DB 401 as the culture inspection time in correspondence with the work ID 71 and the culture medium ID 65 thus specified. Similarly to the culture start time, a preset time may be used as the culture inspection time on the premise that the registration processing of the culture medium information is executed at that time.

On the other hand, the registration processing unit 421 makes the colony detection determination unit 422 detect the number of colonies in the culture medium image data based on the culture medium image data included in the culture medium information, and registers the detected number of colonies with the culture medium information DB 401 in correspondence with the line ID and the culture medium ID 65 for which the detected number of colonies is specified.

Then, the registration processing unit 421 registers the acceptance/rejection determination result of the specimen of each film-type culture medium 60, determined by the colony detection determination unit 422 based on the number of colonies, with the culture medium information DB 401.

The registration processing unit 421 may register the received culture medium image data with the culture medium information DB 401 as the culture medium image data as it is, or may additionally register the culture medium image data generated (i.e., re-drawn) at time of detecting the number of colonies with the culture medium information DB 401.

### Colony detection determination unit

Next, the detail of the colony detection determination unit 422 in the server device 40 of this embodiment will be described.

The colony detection determination unit 422 detects the number of colonies cultured in the specimen in the culture medium image data based on the culture medium image data included in the culture medium information, and executes the determination as to whether the specimen in each film-type culture medium 60 is normal or abnormal in view of sanitary management, based on the number of colonies thus detected.

### (Detecting number of colonies)

The colony detection determination unit 422 executes the detection of the number of the colonies based on each culture medium image data, at the time of registering the culture medium information with the culture medium information DB 401 or at a predetermined timing, under the control of the registration processing unit 421, and supplies the number of colonies to the registration processing unit 421.

Specifically, the colony detection determination unit 422 detects, in the culture medium area of the culture medium image data, the pixels whose pixel value is equal to or larger than a predetermined value. For example, the colony detection determination unit 422 reads out a maximum pixel value and a minimum pixel value determined in advance in each sub-pixel of R, G and B, and detects the pixels whose pixel values of the sub-pixels are within the range of the maximum value and the minimum value. The maximum value and the minimum value in each sub-pixel of R, G and B may be predetermined by a manager, or may be predetermined according to the types of bacteria or the dilution rates.

Also, the colony detection determination unit 422 executes the image filtering processing to reduce noise. For example, the colony detection determination unit 422 executes the expansion processing which replaces all the pixels neighboring the pixel detected (hereinafter referred to as "the detected pixel") with the detected pixel when there exists another detected pixel neighboring to the detected pixel, and executes the contraction processing which replaces all the pixels neighboring a non-detected pixel when there exists the non-detected pixel neighboring to the detected pixel.

Then, the colony detection determination unit 422 executes labeling which connects the neighboring detected pixel with the pixel recognizedas the detectedpixel by the image filterprocessing, recognizes the connected group of the detected pixels as one object, i.e., a colony, and calculates the centroid of each recognized colony to detect the number of coordinates as the number of colonies. Additionally, the colony detection determination unit 422 supplies the detected number of colonies to the registration processing unit 421.

The colony detection determination unit 422 may re-draw the culture medium image data based on the detected number of colonies and the coordinates of each colony, and supply the re-drawn culture medium image data to the registration processing unit 421.

### (Acceptance/Rejection determination of specimen)

The colony detection determination unit 422 executes the culture medium determination as to whether the specimen in each film-type culture medium 60 is normal or abnormal (i.e., the acceptance/rejection determination of the specimen). For example, the registration processing unit 421 determines the acceptance/rejection of each specimen based on the threshold value (i.e., the criterion) of number of bacteria predetermined for each type of bacterium to be detected from the specimen, and supplies the result to the registration processing unit 421.

Particularly, since the criterion is different based on the bacterium type and/or the dilution rate in this embodiment, the registration processing unit 421 reads out the culture medium determination criterion information from the ROM/RAM 440 based on the criterion of culturing the specimen and the bacterium type to execute the culture medium determination.

Each determination criterion information is specified in correspondence with the culture medium ID 65. Namely, since the type of the film-type culture medium 60 is different between the types of bacteria detected from the specimen, the type of bacterium to be detected can be specified by specifying the culture medium ID 65. Accordingly, in this embodiment, the determination criterion information specified based on the culture medium ID 65 can be read out by predetermining the dilution rate.

Also, in this embodiment, since the acceptance/rejection determination of each film-type culture medium 60 is used in the process determination, the line determination and the lot determination, the culture medium determination is executed in correspondence with each of those determination. The method of determining the acceptance/rejection of each film-type culture medium 60 in the process determination, the line determination and the lot determination will be described later.

### Lot determination processing unit

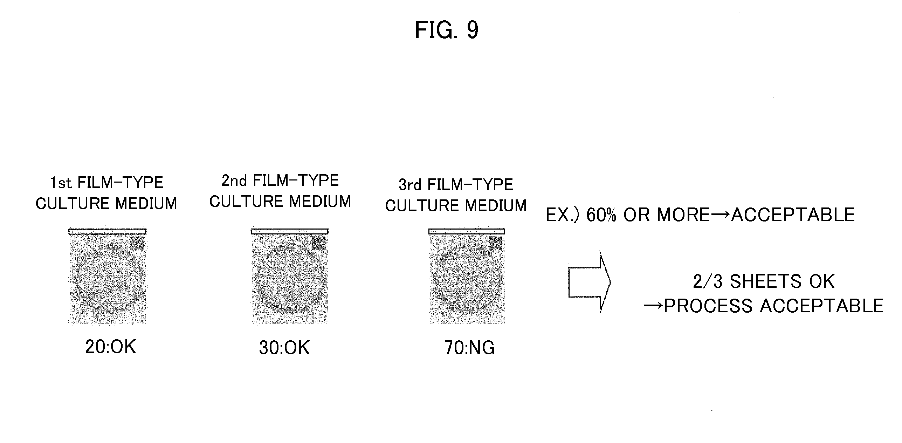

Next, the detail of the lot determination processing unit 423 in the server device 40 of this embodiment will be described with reference to FIGS. 9 to 11. FIGS. 9 to 11 are diagrams for explaining the process determination in the server device 40 of this embodiment.

The lot determination processing unit 423 of this embodiment executes data analysis related to the sanitary management in the lot, the work line 80 or the process (i.e., the acceptance/rejection determination) based on the instruction of the manager inputted to the manager terminal device 20.

Specifically, the lot determination processing unit 423 determines whether or not the food group produced or inspected in the same condition satisfies a predetermined condition, for each lot, based on the line information of the work line 80 used for the corresponding lot and the culture medium information (at least the number of colonies) registered in correspondence with the corresponding work line 80.

Specifically, when the lot determination is instructed, the lot determination processing unit 423 executes the line determination of the work line 80 which determines whether or not the work line 80 included in the corresponding lot and the process belonging to the work line 80 are normal or abnormal in view of the sanitary management (i.e., the acceptance/rejection determination of the sanitary management) and the process determination of each process (i.e., the acceptance/rejection determination of sanitary management), and executes the lot determination using the process determination and the line determination.

Particularly, the lot determination processing unit 423 determines the acceptance/rejection of the process in view of the sanitary management based on the number of colonies in the film-type culture medium 60 detected from the process and/or the acceptance/rejection determination of the specimen, determines the acceptance/rejection of the work line 80 in view of the sanitary management based on the acceptance/rejection determination of the process included in the corresponding work line 80, and determines the acceptance/rejection of the lot in view of the sanitary management based on the acceptance/rejection determination of the work line 80 included in the corresponding lot.

The lot determination processing unit 423 of this embodiment may execute, not the lot determination, but only the process determination or the line determination as the data analysis.

### (Process determination)

The lot determination processing unit 423 executes each processing determination based on the culture medium information of plural film-type culture media 60 registered in correspondence with the corresponding process. Namely, the lot determination processing unit 423 executes each process determination by a total determination based on each determination results of the plural film-type culture media 60.

Specifically, the lot determination processing unit 423 obtains the culture medium determination result of the corresponding film-type culture medium 60 (i.e., all the culture medium determination results registered in the culture medium information having the process ID 72 of the process for which the process determination should be executed). The lot determination processing unit 423 determines that the process to be determined is acceptable when the number of the acceptance/rejection determination results satisfies the predetermined condition, and determines that the process to be determined is rejected when the number of the acceptance/rejection determination results does not satisfy the predetermined condition.

For example, as shown in FIG. 9, it is assumed that three film-type culture media 60 have the process ID 72 of the process to be determined, the number of colonies of the first film-type culture medium 60 is "20", the number of colonies of the second film-type culture medium 60 is "30", the number of colonies of the third film-type culture medium 60 is "70", and the predetermined condition is that the the process is determined to be acceptable if the process includes the acceptable culture medium information of "60%". In this case, two determination results of three film-type culture media 60 are acceptable, and therefore the lot determination processing unit 423 determines that this process is acceptable.

Also, instead of the above method, the lot determination processing unit 423 may execute the process determination of the process to be determined based on the number of colonies included in the culture medium information having the process ID 72 of the process for which the process determination is to be executed.

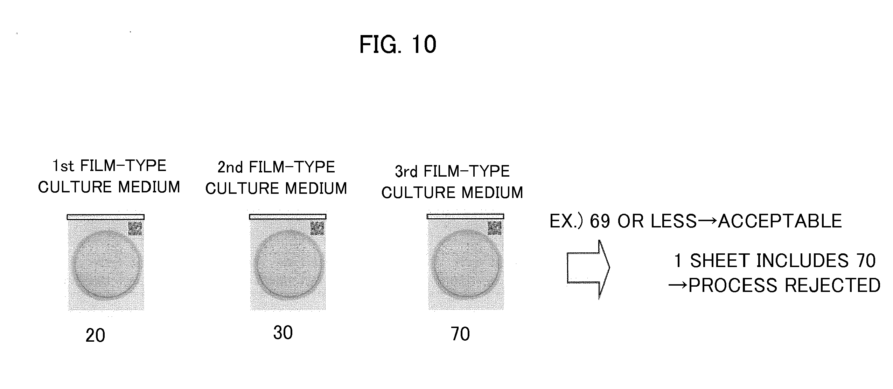

For example, as shown in FIG. 10, it is assumed that three film-type culture media 60 have the process ID 72 of the process to be determined, the number of colonies of the first film-type culture medium 60 is "20", the number of colonies of the second film-type culture medium 60 is "30", the number of colonies of the third film-type culture medium 60 is "70", and the predetermined condition is that the process is determined to be acceptable if the process includes no culture medium information whose number of colonies is equal to or larger than the threshold of the number of colonies "70". In this case, the third film-type culture medium 60 has the number of colonies "70", and therefore the lot determination processing unit 423 determines that this process is rejected.

On the other hand, instead of the above process determination method using the threshold of the number of colonies, the lot determination processing unit 423 may execute the process determination of the process to be determined based on the number of the colonies included in all the culture medium information having the process ID 72 of the process for which the process determination is to be executed.

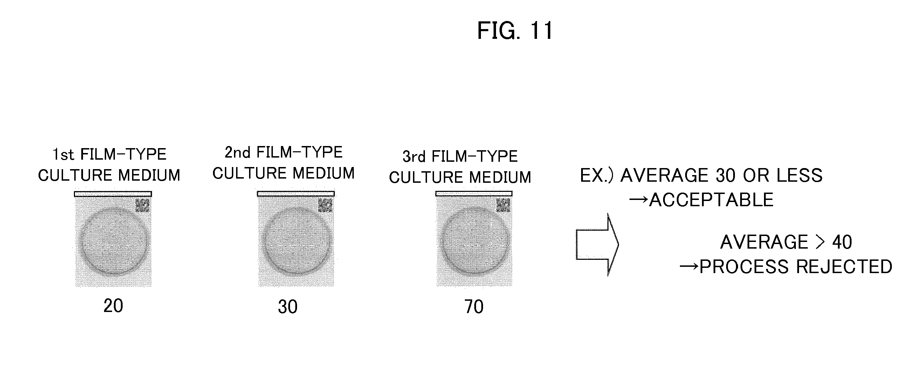

For example, as shown in FIG. 11, it is assumed that three film-type culture media 60 have the process ID 72 of the process to be determined, the number of colonies of the first film-type culture medium 60 is "20", the number of colonies of the second film-type culture medium 60 is "30", the number of colonies of the third film-type culture medium 60 is "70", and the predetermined condition is that the process is determined to be acceptable if the average of all the numbers of colonies is equal to or smaller than the threshold "30". In this case, the average of the numbers of the colonies of three third film-type culture media 60 is "40", and therefore the lot determination processing unit 423 determines that this process is acceptable.

On the other hand, at the time of executing the process determination, the lot determination processing unit 423 may weight the predetermined process based on the position and/or role, and execute the process determination in consideration of the weight.

Specifically, the lot determination processing unit 423 gives a large weight to the determination result of the process position such as the most downstream process (final process), and gives a large weight to the determination result of the process which sterilize the detected bacteria, such as boiling or burning.

For example, in the case of FIG. 9, the lot determination processing unit 423 executes the process determination with doubling the acceptance/rejection determination result of the film-type culture medium 60 in the most downstream process. Namely, in this case, the lot determination processing unit 423 assumes that it inspects two third film-type culture media 60 and obtains the same value, and determines that the process is acceptable if the acceptance rate of the four film-type culture media 60 is equal to or larger than 60%. For example, in the case of FIG. 9, two determination results out of four film-type culture media 60 are acceptable, and therefore the lot determination processing unit 423 determines that the process is rejected.

Also, in the case of FIG. 10, the lot determination processing unit 423 executes the process determination with setting the threshold value at the time of determining the acceptance/rejection of the film-type culture medium 60 in the most downstreamprocess to 1/2. Namely, in this case, the threshold of the number of colonies serving as the acceptance/rejection criterion in the third film-type culture medium 60 becomes 1/2, and the lot determination processing unit 423 determines that the process is acceptable if the number of colonies is smaller than the threshold value in all the three film-type culture media 60. For example, in the case of FIG. 10, the threshold of the third film-type culture medium 60 is "35" and the number of colonies is larger than the threshold. Therefore, the lot determination processing unit 423 determines that the process is rejected.

Further, in the case of FIG. 11, the lot determination processing unit 423 executes the process determination with setting the threshold value at the time of determining the acceptance/rejection of the film-type culture medium 60 in the most downstream process to twice. Namely, in this case, the threshold of the number of colonies serving as the acceptance/rejection criterion in the third film-type culture medium 60 becomes twice, and the lot determination processing unit 423 determines that the process is acceptable if the average of the number of colonies in three film-type culture media 60 is smaller than a predetermined value (e. g., "30"). For example, in the case of FIG. 11, the number of colonies in the third film-type culture medium 60 is "140" and the average is "63.3". Therefore, the lot determination processing unit 423 determines that the process is rejected.

The lot determination processing unit 423 of this embodiment may select one of the above-described methods based on the number of the culture medium information registered in each process, or may execute the process determination by combining two or three of the above methods. The predetermined condition is alterable depending on the type of specimen and type of bacterium to be detected. Also, the predetermined condition may be set by the manager or may be supplied as a part of a program.

### (Line Determination)

The lot determination processing unit 423 executes the line determination of each work line 80 based on the acceptance/rejection determination, in view of sanitary management, of the plural processes registered in correspondence with the corresponding line information. Namely, the lot determination processing unit 423 executes the line determination by a total determination based on each determination result of the plural processes.

Specifically, as described above, the lot determination processing unit 423 obtains the determination result of the process determination of the process including the line ID of the work line 80 for which the line determination is to be executed. The lot determination processing unit 423 determines that the line determination of the work line 80 to be determined is acceptable when the number of the acceptance/rejection of the determination results satisfies the predetermined condition, and determines that the line determination of the work line 80 to be determined is rejected when the number does not satisfy the predetermined condition.

For example, in a case where three processes have the line ID of the work line 80 to be determined, if the first process is "acceptable", the second process is "acceptable", the thirdprocess is "rejected", and the predetermined condition is that the work line 80 is acceptable if it includes "60%" acceptable processes, the lot determination processing unit 423 determines that the line determination of the work line 80 to be determined is acceptable.

### (Lot Determination)

The lot determination processing unit 423 executes the lot determination of each lot based on the acceptance/rejection determination, in view of sanitary management, of the plural work lines 80 registered in correspondence with the corresponding lot information. Namely, the lot determination processing unit 423 executes the lot determination by a total determination based on each determination result of plural work lines 80.

Specifically, the lot determination processing unit 423 executes the lot determination of the lot to be determined based on the acceptance/rejection determination of the sanitary management information of the work line 80 having the lot ID of the lot for which the lot determination is to be executed.

For example, when the predetermined condition is that the lot determination processing unit 423 determines that the lot is acceptable if the lot is constituted by the accepted work line 80 of "60%", it is assumed that three work lines 80 has the lot ID of the lot to be determined. In this case, if the first work line 80 is "acceptable", the second work line 80 is "acceptable" and the third work line 80 is "rejected", the lot determination processing unit 423 determines that the lot determination of the lot to be determined is acceptable.

If the work time of the lot is long, the lot determination maybe executed by dividing the time into predetermined time periods (e.g., 8 hours or 12 hours). In this case, when the lot determination is executed while the work is being interrupted, a dummy result may be used or the determination can be executed during the interruption of the work.

### (Others)

The lot determination processing unit 423 can execute determination and data analysis other than those described above by using the culture medium information, the line information, the lot information and the work management DB, and can supply the determination result or the data analysis result to the manager terminal device 20 via the report processing unit 424 in such a manner that the manager can browse.

Specifically, the lot determination processing unit 423 executes the predetermined analysis of the culture medium information or the culture medium image data in one culture medium ID in time series from the culture start time to the end of culture, in order to confirm reliability of the specimen cultured in each film-type culture medium 60, i.e., in order to confirm whether or not the inspection error is happening.

For example, the lot determination processing unit 423 extracts plural culture medium information having a specific culture medium ID (the same culture medium ID) from the culture start time to a predetermined time, together with the culture medium image data, based on the instruction by the manager received via the manager terminal device 20. Then, the lot determination processing unit 423 executes the predetermined analysis of each culture medium information and each culture medium image data along a predetermined time series, or aggregates each culture medium information and each image data in time series. Also, the lot determination processing unit 423 makes the report processing unit 424 generate browsing data of the analysis result or the aggregation result in a predetermined format and supply the generated browsing data to the manager terminal device 20 in a manner browsable by the manager.

At this time, in cooperation with the report processing unit 424, the lot determination processing unit 423 may make each culture medium information in a culture medium ID individually browsable for a statutory sanitary management report based on the instruction by the manager terminal device 20.

The lot determination processing unit 423 may have a search function of the culture medium information, the line information, the lot information or the work management information. Specifically, the lot determination processing unit 423 may search the database 400 by using the lot ID, the work line ID, the process ID, the culture start time, the work date and time of the lot, the culture medium ID and the type of specimen as a search key, and cooperates with the report processing unit 424 to generate data of the corresponding culture medium information and various information in a manner browsable by the manager.

The report processing unit 424 may supply information related to the culture medium information (the culture medium image data, the number of colonies and the culture medium determination result), the process information, the line information or lot specified by the search in a predetermined report format, based on the instruction by the manager received via the manager terminal device 20. Namely, the report processing unit 424 can supply various kinds of information in a case of a statutory inspection report or else as the proof or the report.

### Report processing unit

Next, the report processing unit 424 in the server device 40 of this embodiment will be described.

The report processing unit 424 generates the analysis result (i.e., the determination result of acceptance/rejection) of the data analysis related to the sanitary management in the designated lot, work line 80 or process as report data, based on the instruction by the manager inputted to the manager terminal device 20 and template data having a predetermined report format, and supplies the generated report data to the manager terminal device 20 via the communication control unit 410 in a manner browsable by the manager.

Specifically, the report processing unit 424 extracts necessary information such as the culture medium image data, the number of colonies or the culture medium determination result from the culture medium information, the line information and the lot information, and assigns each information to the template data based on the extracted information and the analysis result obtained by the lot determination processing unit 423 to generate the report data. Then, the report processing unit 424 supplies the generated report data to the manager terminal device 20.

Also, as described above, the report processing unit 424 can generate the predetermined report data or browsing data in accordance with various analysis or search, and supplies them to the manager terminal device 20. Namely, the report processing unit 424 can supply the culture medium information, the process information, the line information or information related to the lot obtained by the search in a predetermined report format, and can supply various information in a statutory inspection report or else as its proof or report.

### [6] Operation processing of sanitary management system

### Registration processing of culture medium information

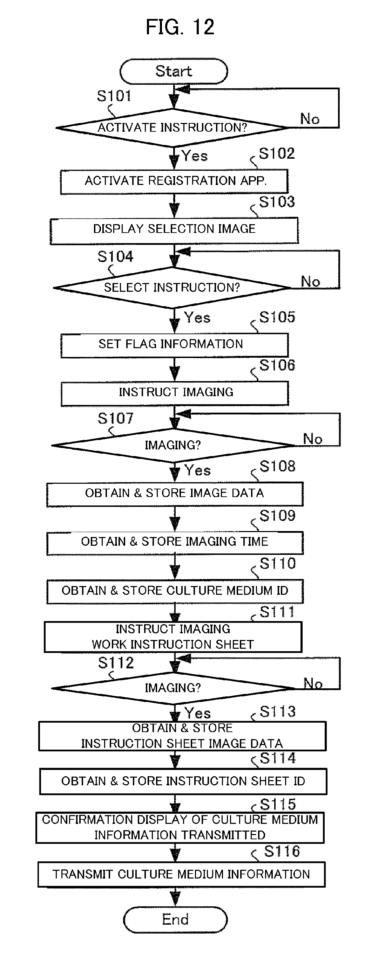

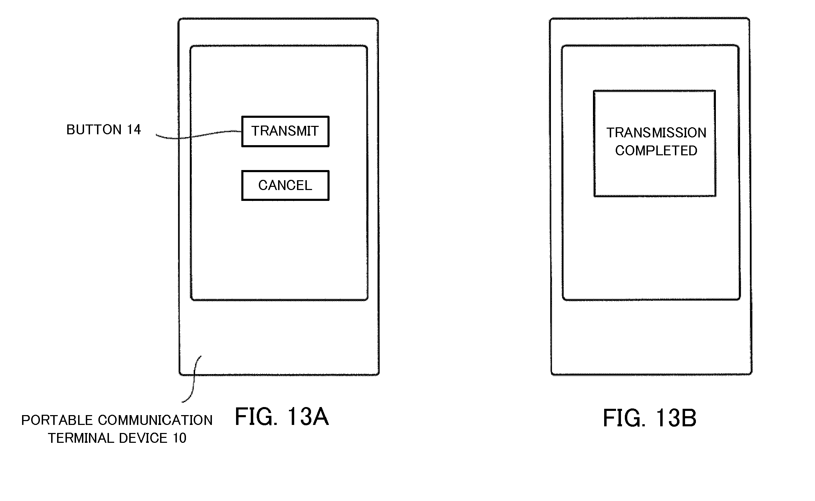

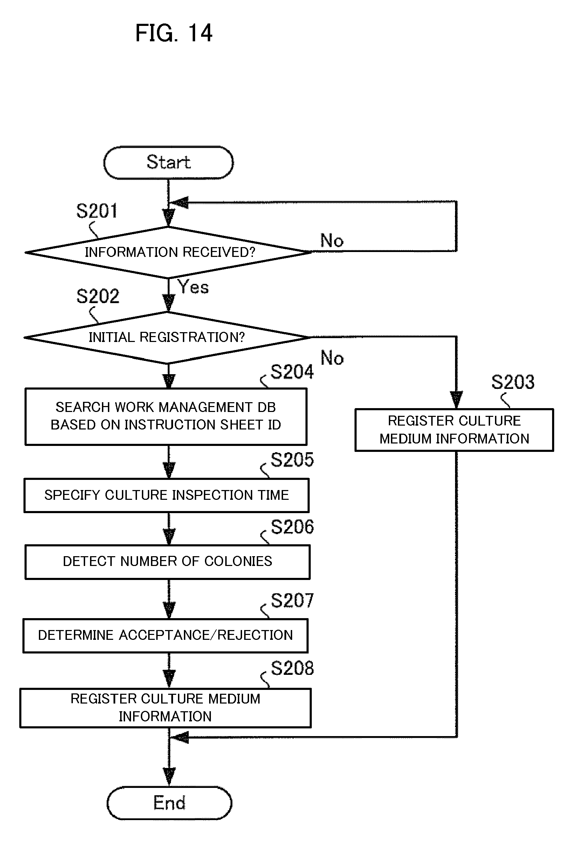

Next, description will be given of registration processing of the culture medium information in the portable communication terminal device 10 and the server device 40 of this embodiment with reference to FIGS. 12 to 14. FIG. 12 is a flowchart showing an operation of the registration processing of the culture medium information in the portable communication terminal device 10 of this embodiment, and FIGS. 13A and 13B are examples of the image displayed on the display unit 160 when the culture medium information is transmitted by the portable communication terminal device 10. FIG. 14 is a flowchart showing an operation of the registration processing of the culture medium information in the server device 40 of this embodiment.

In this operation, it is assumed that the line information DB 402 and the lot information DB 403 already store the lot information and the line information in correspondence with the work instruction sheet 70. It is also assumed that the film-type culture medium 60 and the work instruction sheet 70 are provided with the culture medium ID or the instruction sheet ID in a form of two-dimensional barcode. Further, in this operation, it is assumed that the detection of the number of colonies in each film-type culture medium 60 is executed when the culture medium information is registered with the database 400.

First, in the portable communication terminal 10, when the application control unit 120 detects an activation instruction of the culture medium registration application via the operation unit 170 (step S101), it reads out the culture medium registration application from the application storage unit 101 and activates it (step S102). At this time, the application control unit 120 executes necessary processing such as an initialization of the work memory and else under the control of the terminal control management unit 190.

Next, the application control unit 120 cooperates with the display control unit 161 to display the image for selecting one of the initial registration of the film-type culture medium 60 or the post-culture registration after the passage of the predetermined time from the start of culture, on the display unit 160, and waits for the input (step S103). For example, the application control unit 120 cooperates with the display control unit 161 to display the image shown in FIG. 5 on the display unit 160.

Next, when the application control unit 120 detects the input of the selection instruction by the operation unit 170 (step S104), it determines whether it is the initial registration or the post-culture registration and sets the result to the flag information (step S105).

Next, the application control unit 120 displays the image suggesting the imaging of the film-type culture medium 60 on the display unit 160, and cooperates with the operation unit 170 to wait for the imaging by the image data generation unit 110 (step S106). For example, the application control unit 120 cooperates with the display control unit 161 to display the image shown in FIG. 6A on the display unit 160.

Next, the application control unit 120 cooperates with the image data generation unit 110, the display control unit 161 and the operation unit 170 to detect the imaging by the image data generation unit 110 (step S107), obtain the culture medium image data of the film-type culture medium 60 imaged by the image data generation unit 110 together with the culture medium ID 65, and store it in the image data storage unit 102 with the predetermined image ID (step S108).

Next, the application control unit 120 obtains the current time from the timer 180 as the imaging time of the culture medium image data, and stores it in the image data storage unit 102 as the metadata in correspondence with the culture medium image data (step S109).

Next, the application control unit 120 analyses the two-dimensional barcode formed on the predetermined area of the obtained culture medium image data to obtain the culture medium ID 65, and stores it in the image data storage unit 102 in correspondence with the culture medium image data (step S110).

Next, the application control unit 120 cooperates with the display control unit 161 to display the image for obtaining the instruction sheet ID, the process ID and the instruction sheet image data on the display unit 160 and wait to obtain the instruction sheet image data (step S111). For example, the application control unit 120 cooperates with the display control unit 161 to display the image shown in FIG. 7A on the display unit 160.

Next, the application control unit 120 cooperates with the image data generation unit 110, the display control unit 161 and the operation unit 170 to detect the imaging by the image data generation unit 110 (step S112), obtain the instruction sheet image data imaging in which the work ID 71 is imaged, and store it in the image data storage unit 102 in correspondence with the culture medium image data obtained in step S108 (step S113).

When obtaining the process ID 72 as the work ID 71 or obtaining one process ID 72 from plural process IDs 72, the application control unit 120 cooperates with the display control unit 161 and the operation unit 170 to execute a display to make the user select the process ID to be obtained, and obtain the corresponding process ID 72.

Next, the application control unit 120 analyses the two-dimensional barcode formed on the predetermined area of the imaged instruction sheet image data to obtain the work ID 71, and stores it in the image data storage unit 102 in correspondence with the instruction sheet image data (step S114).

Next, the application control unit 120 cooperates with the display control unit 161 and the operation unit 170 to make the worker confirm the culture medium image data, the culture medium ID 65, the instruction sheet image data, the work ID 71 and the imaging time thus obtained (step S115). The application control unit 120 may obtain the culture medium image data and the instruction sheet image data again, based on the instruction by the worker, at the time when the worker confirms the obtained culture medium information. Also, the application control unit 120 may cooperate with the display control unit 161 and the operation unit 170 to permit the worker to correct each image data and culture medium information.

Next, the application control unit 120 transmits the obtained image data, its metadata and the flag information as the culture medium information to the server device 40 via the network communication unit 130, together with the flag information indicating one of the initial registration and the post-culture registration, the terminal ID and the work instruction ID, based on the instruction by the worker (step S116), and then ends the registration processing by the portable communication terminal device 10.

At this time, the display control unit 161 displays the image shown in FIGS. 13A and 13B on the display unit 160. Also, the network communication unit 130 executes login for the access to the server device 40 based on the terminal ID and/or the inputted ID and the password at the time of transmitting the culture medium information, and establishes the communication line with the server device 40 after the completion of the login to transmit the culture medium information.

On the other hand, in the server device 40, when the communication control unit 410 receives the work ID 71and the culture medium information transmitted from the portable communication terminal device 10 (step S201), the registration processing unit 421 determines whether it is the initial registration or the post-culture registration based on the flag information included in the received culture medium information (step S202). At this time, the process goes to step S203 when it is determined to be the initial registration, and the process goes to the step S204 when it is not determined to be the initial registration, i.e., the post-culture registration.

Next, when the registration processing unit 421 determines the initial registration, it registers the culture medium information with the culture medium information DB 401, based on the work ID 71, in correspondence with the line related information such as the lot information and the line information (step S203), and ends the operation. While the culture start time is stored in the workmanagement DB 404 in advance, the registrationprocessing unit 421 may register the imaging time included in the received culture medium information with the work management DB 404 as the culture start time in the process of step S203.

On the other hand, when the register processing unit 421 determines the post-culture registration, it searches the work management DB 404 based on the obtained work ID 71 (step S204), specifies the line related information such as the lot information having the work ID 71 and the line information of the work line 80 from which the specimen is to be extracted, and specifies the culture inspection time for registering the culture medium information based on the imaging time included in the culture medium information (step S205).

At this time, the registration processing unit 421 may cooperate with the portable communication terminal device 10 to make the portable communication terminal device 10 designate the culture inspection time for registering the culture medium information and register it, or may register the imaging time included in the received culture medium information as it is as the culture inspection time.

Also, the registration processing unit 421 may manage the culture medium registration at the time of the culture inspection time already executed, and specify the culture medium inspection time based on the timing of receiving the culture medium information and the culture inspection time already used for the registration of the culture medium information. Namely, the registration processing unit 421 may rewrite the execution flag every time the registration of the culture medium information is executed, and specify the culture inspection time in comparison with the received imaging time, by referring to the information of the execution flag.

Next, the registration processing unit 421 makes the colony detection determination unit 422 detect the number of the colonies of the specimen (step S206). Specifically, the colony detection determination unit 422 analyses the predetermined area of the culture medium image data and detects the number of colonies.

Next, the colony detection determination unit 422 reads out the culture medium determination criterion based on the type of the specimen and the bacterium to be detected which are specified based on the work ID 71, and determines whether or not the detected number of colonies satisfies the predetermined condition (step S207). Namely, the colony detection determination unit 422 determines the acceptance (normal) when the predetermined condition is satisfied, and determines the rejection (abnormal) when the predetermined condition is not satisfied.

For example, the colony detection determination unit 422 reads the culture medium determination criterion based on the specified line information, and determines the acceptance when the detected number of colonies is equal to or smaller than the threshold value given by the culture medium determination criterion, and determines the rejection when the detected number of colonies is larger than the threshold value.

Instead of the above automatic registration, the colony detection determination unit 422 may cooperate with the portable communication terminal device 10 to display the detected number of colonies and the acceptance/rejection determination result on the display unit 160 of the portable communication terminal device 10 so that the user can be browse and confirm them.

Next, the registration processing unit 421 registers the determined acceptance/rejection, the detected number of colonies and the culture start time information having the imaging time included in the culture medium information as the culture start time with the culture medium information DB 401, together with the recognized culture medium ID 65, in correspondence with the specified work ID 71 (step S208), and ends this operation.

The registration processing unit 421 displays may make the portable communication terminal device 10 display the line information and information to be registered on the display unit 160 and register the culture medium information based on the user instruction inputted via the portable communication terminal device 10.

Also, in a case where the portable communication terminal device 10 transmits the culture medium image data and the instruction sheet image data and the server device 40 analyses each image data to recognize the culture medium ID 65 and the work ID 71, the registration processing unit 421 analyses the work ID 71 and the culture medium ID 65 imaged on the predetermined area of the instruction sheet image data and the culture medium image data thus received to obtain the work ID 71 and the culture medium ID 65 in step S201.

### Lot determination processing

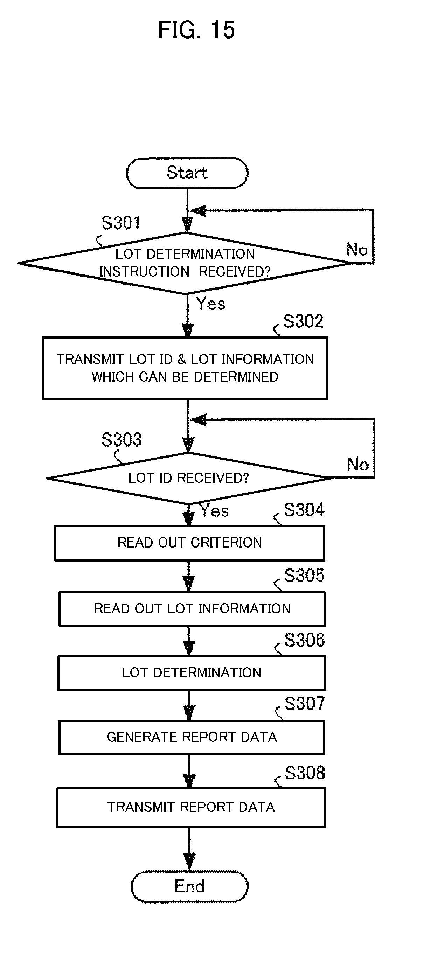

Next, the operation of the lot determination processing in the server device 40 of this embodiment will be described with reference to FIG. 15. FIG. 15 is a flowchart showing the operation of the lot determination processing in the server device 40 of this embodiment.

In this operation, it is assumed that the corresponding culture medium information has been registered and the process determination criterion information, the line determination criterion information and the lot determination criterion information have been registered in advance.

First, when the communication control unit 410 in the server device 40 receives the instruction of the lot determination transmitted from the manager terminal device 20 (step S301), the lot determination processing unit 423 cooperates with the communication control unit 410 to search the lot ID for which the lot determination is possible, transmits the lot ID of the lot for which the lot determination is possible (i.e., the instruction sheet ID) and the corresponding lot information to the manager terminal device 20 such that the manager can browse and select, and then wait for the instruction of the lot ID for which the lot determination process is executed (step S302).

The manager terminal device 20 displays the lot ID of the lot for which the lot determination is possible on a predetermined display screen in a manner browsable and selectable by the manager.

Next, when the communication control unit 410 receives the lot ID specified by the manager via the manager terminal device 20 (step S303), the lot determination processing unit 423 reads out each of the criterion information indicating the condition of the process determination of the process belonging to the lot, the line determination and the lot determination from the ROM/RAM 440 (step S304).

Next, the lot determination processing unit 423 reads out the various lot information, the line information and the culture medium information of the corresponding lot ID (step S305), and execute the lot determination with executing the process determination and the line determination, based on the lot information, the line information and the culture medium information thus read out (step S306). Specifically, the lot determination processing unit 423 executes the process determination of each process specified by the lot information, and executes the line determination of each work line 80 specified by the lot information based on the process determination result of the process determination. Then, the lot determination processing unit 423 executes the lot determination based on the line determination result.

Next, the lot determination processing unit 423 reads out the predetermined template serving as the report form of the determination, and assigns the lot determination result to the template to generate the report data (step S307).

Finally, the lot determination processing unit 423 cooperates with the communication control unit 410 to supply the generated report data to the manager terminal device 20 (step S308), and ends this operation.

When receiving the report data, the manager terminal device 20 displays it in a manner browsable by the manager. However, the lot determination processing unit 423 may transmit, not the report data, but print data printable by a printer to the manager terminal device 20.

### [7] Modified examples

### 1st Modified example

In this embodiment, in a case where plural processes or plural work lines 80 exist, instead of specifying the process or the work line 80 from which the specimen is extracted based on the instruction by the worker to specify the work ID 71, the process or the work line 80 may be specified by using the GPS function or other current position detection function in the portable communication terminal device 10, or an area (hereinafter referred to as "the extraction area") in the process or the work line 80 from which the specimen is extracted may be specified.

Specifically, when the position of imaging the film-type culture medium 60 coincides with the position or the process from which the specimen is extracted, in the portable communication terminal device 10, the application control unit 120 makes the current position detection unit 150 detect the current position at the time of extracting the culture medium image, and transmits the detected position information or the area to which the position belongs, to the server device 40, as the extraction area together with the culture medium information.

In the server device 40, the registration processing unit 421 compares the position information with the position of the work line 80 stored beforehand in the line information or the position information of the process position, and specifies the work line 80 or the process. In this case, the registration processing unit 421 constitutes the process information obtaining unit of the present invention, for example.

In the server device 40, the registration processing unit 421 may register the extraction area from which the specimen is extracted in each process as the culture medium information, and the lot determination processing unit 423 may execute the process determination, the line determination and the lot determination for each extraction area or based on the culture medium information equally obtained from each extraction area.

For example, the registration processing unit 421 may specify the extraction areas such as a first part of process, a middle part of process and a last part of process based on the position information, or such as a left side, a center and a right side of the physical area in the work line 80 in which food is flowing. The lot determination processing unit 423 may execute the process determination of the first part of process, the middle part of process and the last part of process, or the left side, the center and the right side, or execute the process determination by obtaining the culture medium information of the first part of process, the middle part of process and the last part of process, or the left side, the center and the right side (so that the number of the film-type culture medium used for the determination becomes equal).

### 2nd Modified example

In this embodiment, instead of executing the registration processing of the culture medium information by using the portable communication terminal device 10 such as the tablet-type information terminal device, the smartphone or the portable telephone, the registration processing of the culture medium information may be realized by using a personal computer of laptop type or desktop type, and an image input device such as a scanner, a digital camera or a smartphone.

In this case, the personal computer and the image input device may be connected by a predetermined communication standard to integrally use the personal computer and the image input device to realize the registration processing of the culture medium information. Alternatively, the culture medium image data and the instruction sheet image data obtained in advance may be stored in a physical memory such as a memory card and the like, and each image data thus stored may be read out by the personal computer to realize the registration processing of the culture medium information.

### 3rd Modified example

In this embodiment, at the time of obtaining and registering each culture medium information, purpose type information indicating a type of purpose of using the culture medium information may be included in the culture medium information, and the culture medium determination, the process determination, the line determination and the lot determination may be executed based on the purpose type information.

Specifically, in the portable communication terminal device 10, the application control unit 120 obtains information of intended use of the culture medium to be registered as the purpose type information, and transmits it to the server device 40 as a part of the culture medium information. For example, the purpose type information includes information of the intended use at the time of inspecting the specimen, such as the preliminary inspection, the main inspection or the re-inspection.

In the server device 40, the registration processing unit 421 registers the purpose type information included in the obtained culture medium information with the culture medium DB 401 in correspondence with each culture medium ID 65.

On the other hand, when executing the normal inspection (the main inspection), the lot determination processing unit 423 executes various determination by using the culture medium information indication the normal inspection (the main inspection). When the registration error, read-out error or data loss of the culture medium information of the film-type culture medium 60 for the normal inspection occur, the lot determination processing unit 423 supplements the data by the culture medium information indicating the preliminary inspection to execute various determination.

When the determination result of each determination is determined to be abnormal (rejected) and re-determination (i.e., re-inspection) is performed, the lot determination processing unit 423 executes the re-determination to improve the reliability based on the culture medium information indicating the re-inspection or based on the culture medium information indicating the re-inspection in addition to the culture medium information of the main inspection.

In this case, the culture medium DB 401 stores the purpose type information by the flag information in correspondence with each culture medium ID 65, and the lot determination processing unit 423 reads out the flag information to obtain necessary culture medium information from the culture medium information DB 401 based on the instruction by the manager.

Also, in this case, information may be registered to enable the use of the registered culture medium information according to the purpose, the data management can be executed to use the information for various data analysis and to improve the reliability of the data analysis.

### 4th Modified example

In this embodiment, the portable communication terminal device 10, the manager terminal device 20 and the server device 40 may be placed and used in the same site, or may be separately placed at remote locations such as places outside the country, or may be used at the remote locations to execute each processing described above. However, it is the premise that the portable communication terminal device 10 is used in the same lot.

### 5th Modified example

In this embodiment, the culture medium information in the specimen cultured in the film-type culture medium 60 is registered. However, the culture medium information of the specimen cultured in an agar medium may be registered.

In this case, the culture medium ID 65 such as a two-dimensional barcode is issued for each agar medium, and the culture medium ID 65 is attached to a petri dish of the agar medium. The portable communication terminal device 10 obtains the culture medium ID 65 by imaging the culture medium ID 65 attached to the dish. In this case, when the IC tag storing the culture medium ID 65 is used, it is sufficient that the IC tag is formed on the dish.

### 6th Modified example

In this embodiment, instead of obtaining each of the culture medium ID 65 and the instruction sheet ID 71 by imaging the culture medium ID 65 or the work ID 71 by the image data generation unit 110, the worker may input the culture medium ID 65 and the work ID 71.

In this case, the application control unit 120 cooperates with the display control unit 161 and the operation unit 170 to make the worker directly and manually input the culture medium ID 65 and the work ID 71 via the operation unit 170.

The application control unit 120 controls the display control unit 161 and the operation unit 170 to enable the selection of the displayed items in a pull-down menu by a click or touch selection, and makes the worker input the culture medium ID 65 and the work ID 71.

In this case, the operation unit 170 constitutes a culture medium information input unit and a work information input unit.

### 7th Modified example

In this embodiment, instead of registering the culture medium information and the work ID 71 with the database 400 when them are transmitted to the server device 40, the portable communication terminal device 10 may have a simple database similar to the database 400 provided in the server device 40 and obtain the culture medium information by referring to the work contents and other various information in the database.

In this case, the application control unit 120 compares each information with the information in the culture medium information DB, the line information DB and the work management DB provided in the portable communication terminal device 10 and displays whether or not the culture medium to be obtained is correct or not on the display unit 160, thereby to obtain each culture medium information.

Without providing a simple database in the portable communication terminal device 10, the application control unit 120 may cooperate with the server device 40 to compare with the line information DB 402, the lot information DB 403 and the work management DB 404 to execute and obtain the input of each culture medium information.

### 8th Modified example

In this embodiment, instead of supplying the lot determination result to the manager terminal device 20 by the report data, only the result of the process determination, only the result of the line determination, only the result of the specific process determination, only the result of the specific line determination or their combination may be supplied to the manager terminal device 20 by the report data.

In this case, the lot determination processing unit 423 assigns the determination result of the specified process or work line 80 to the report data and transmits it to the manager terminal device 20.

The lot determination processing unit 423 assigns the determination result to the report data to supply it to the manager terminal device 20. However, when the abnormality (rejection) is determined in the process determination result, the line determination result or the lot determination result, it is possible to send a warning indicating that an abnormality exists in the lot or that the re-inspection is needed to the manager, the worker or other user by an e-mail, sound or light, regardless of whether or not the report data is supplied.

In this case, the lot determination processing unit 423 transmits the e-mail registered in advance, or operates the alarm at the place registered in advance. Particularly, as the warning by the e-mail, only the contents indicating the abnormality in the process, the work line or the lot maybe noticed, or the contents suggesting the re-inspection may be noticed.

### 9th Modified example

In this embodiment, the server device 40 detects the number of colonies in the specimen to execute the culture medium determination. However, the portable communication terminal device 10 may be provided with a program for detecting the number of colonies and a program of culture medium determination to execute the one or both of detecting the number of colonies and determination of the culture medium.

In this case, the application control unit 120 transmits the detected number of colonies and the culture medium determination result to the server device 40 as the culture medium information.

### 10th Modified example

In this embodiment, various processing is executed by a single server device 40. However, various processing may be executed by a server system including plural server devices.

### 11th Modified example

In this embodiment, when the culture medium image data is imaged, basically the culture medium ID 65 formed at a part of the film-type culture medium 60 is obtained as the image at the same time. However, the culture medium ID 65 may be inputted by the worker or the manager or may be obtained as the image to be recognized similarly to the above embodiment at the timing of the registration or at a predetermined timing when the culture medium image data is obtained. Also, when the agar medium is used as described above, the culture medium ID 65 may be attached to the petri dish. When the IC tag is used, the IC tag storing the culture medium ID 65 may be attached to the film-type culture medium or other culture media.

As described above, since the sanitary management system S of this embodiment can cooperate with the portable communication terminal device 10 to register the culture medium ID 65 with the database 400 in correspondence with the line related information, the culture medium information and the line related information may easily be registered in correspondence with each other, in comparison with the conventional case where the line related information necessary to produce the report of the film-type culture medium 60 is directly and manually inputted by the worker in a predetermined space of the film-type culture medium or a container of the culture medium.

Therefore, the sanitary management system S of this embodiment can eliminate the complicated work of registering each information to prevent the registration error, and can register the culture medium information to make the data management easy.

Additionally, since the sanitary management system S of this embodiment can register the image data of the film-type culture medium 60 with the database 400 in correspondence with the culture medium identification information and the line related information, the number of colonies can be detected by the predetermined image analysis of the culture medium image data and its information can be also registered.

Therefore, since the sanitary management system S of this embodiment can automate the analysis of each film-type culture medium 60 for registration, it is possible to easily register the information enabling not only the evaluation of the specimen cultured in each culture medium but the automatic determination of the sanitary condition of the process, the work line 80 or the lot.

Additionally, since the sanitary management system S of this embodiment can register the start time of the culture of the specimen and the imaging time of the culture medium image data with the database 400 in correspondence with the culture medium ID 65 and the line related information, the culture medium image varying with the passage of time, i.e., at the culture start time, 24 hours, 48 hours or more from the start of the culture can be registered with the database 400 as the culture medium image data together with the culture medium information.

Therefore, when the culture condition of the specimen is inspected at different time in a single film-type culture medium 60, the sanitary management system S of this embodiment can prevent the erroneous registration of the culture medium information, can easily manage the complicated information of the culture medium, and can register the culture medium information such that the culture medium information can be easily confirmed in time series after the registration.

Additionally, in the sanitary management system S of this embodiment, the culture medium identification information is formed by a barcode such as a two-dimensional barcode or the characters at the container, the packaging material, the blank space of the marking for management or other parts for managing the film-type culture medium itself. Accordingly, at the time of imaging the film-type culture medium 60, the culture medium ID 65 imaged together with the film-type culture medium can be recognized. Thus, the input operation by the worker can be simplified, and the culture medium information can be registered with identifying it from other culture medium information.

Additionally, since the sanitary management system S of this embodiment can register various information necessary at the time of determining the sanitary condition of the work line 80, as the line related information, in correspondence with the culture medium information, it is possible to easily register information that can be used for various analysis such as the sanitary condition of the work line 80.

Additionally, if the image data of the film-type culture medium 60 is registered, the sanitary management system S of this embodiment can determine whether or not the food group (i.e., the lot) produced or inspected under the same condition is abnormal or normal, and therefore the work for evaluation related to the sanitary management of the food group can be automated.

Therefore, the sanitary management system S of this embodiment can eliminate the burden of the complicated work for the worker who manages the lot. Since the determination can be unified, the sanitary management of the food group can be easily and securely executed.

Additionally, since the sanitary management system S of this embodiment can determine the sanitary condition of each film-type culture medium 60 and determine the sanitary condition of the lot, the sanitary condition of the lot can be securely determined. Also, since the the analysis of each film-type culture medium 60 can be automated, the cause of the abnormality found in the food group can be quickly investigated.

Additionally, since the sanitary management system S of this embodiment can specify the process of the specimen cultured on the film-type culture medium or its position at the time of determining the sanitary condition of the film-type culture medium 60, the cause of the abnormality found in the lot can be quickly investigated.

Additionally, since the sanitary management system S of this embodiment can selectively use each criterion for the culture medium determination, the process determination, the line determination and the lot determination from plural criteria based on the specimen to be cultured or the type of bacterium to be detected, it can be used for the sanitary management of various food groups, and it can quickly cope with the change of the work line.

Additionally, since the sanitary management system S of this embodiment can recognize the elapsed time from the culture start time by the imaging time if the culture start time is registered in advance, it is possible to execute the sanitary management of the lot varying with the elapsed time at the culture start time, or at the time 24 hours, 48 hours or more after the start of the culture.

Additionally, in the sanitary management system S, if the abnormality in view of the sanitary management occurs in the lot, it is quickly notified to the manager. Therefore, it is possible to quickly execute the countermeasure such as the stop of shipment.

Additionally, in the sanitary management system S of this embodiment, the registration error can be remarkably reduced by registering the culture medium information with the database 400 andmake the difficult management of the culture mediumeasy, thereby saving the resources without wastefully consuming the culture medium. The sanitary management system s of this embodiment can analyze the sanitary management related to the work line by the electronic data, and supplying the analysis result can be electronically executed. Therefore, resources can be saved in comparison with the case where various data analysis and its supply are performed by using physical resource such as a paper medium.

### INDUSTRIAL APPLICABILITY

This invention can be used for a culture medium information registration system.

### DESCRIPTION OF REFERENCE NUMERALS

- S: Sanitary management system
- 10: Portable communication terminal device
- 20: Manager terminal device
- 30: Network
- 40: Server device
- 60: Film-type culture medium
- 70: Work instruction sheet
- 80: Work line
- 100: Data storage unit
- 101: Application storage unit
- 102: Image data storage unit
- 110: Image data generation unit
- 120: Application control unit
- 130: Network communication unit
- 140: Short-range wireless communication interface
- 150: Current position detection unit
- 160: Display unit
- 170: Operation unit
- 180: Timer
- 190: Terminal management control unit
- 400: Database
- 410: Communication control unit
- 420: Data processing unit
- 421: Registration processing unit
- 422: Colony detection determination unit
- 423: Lot determination processing unit
- 424: Report processing unit
- 430: Server management control unit

## Claims

1. A culture medium information registration system which cooperates with a communication terminal device to register, with a database, culture medium information indicating information of a culture medium on which food produced or inspected by a work line including one or more production process or inspection process is cultured as a specimen, the system comprising:
a culture medium identification information obtaining unit which cooperates with the communication terminal device to obtain culture medium identification information for identification from other culture media;
a work identification information obtaining unit which obtains work identification information which is inputted by the communication terminal device and which identifies a work of the work line;
a search unit which searches line related information related to the work line based on the obtained work identification information, the line related information including information of sanitary management of the work line stored in the database for each work identification information; and
a registration unit which registers the culture medium information including at least the obtained culture medium identification information, with the database, in correspondence with the searched line related information.

2. The culture medium information registration system according to claim 1, further comprising an image data obtaining unit which obtains image data of the specimen imaged by the communication terminal device,
wherein the registration unit registers the culture medium information including at least the obtained image data and the obtained culture medium identification information, with the database, in correspondence with the searched line related information.

3. The culture medium information registration system according to claim 2, further comprising a time information obtaining unit which cooperates with the communication terminal device to obtain time information indicating at least one of a start time of starting culture of the specimen in the culture medium and an imaging time of imaging the image data.

4. The culture medium information registration system according to claim 2 or 3,
wherein the culture medium identification information obtaining unit obtains the culture medium identification information by obtaining the imaged culture medium identification information included in the image data and recognizing the imaged culture medium identification information by image analysis.

5. The culture medium information registration system according to any one of claims 2 to 4, further comprising a detection unit which detects a number of colonies in the specimen included in the image data based on the obtained image data,
wherein the registration unit registers the number of colonies with the database, as the culture medium information, in correspondence with the line related information.

6. The culture medium information registration system according to any one of claims 1 to 5, further comprising a process information obtaining unit which cooperates with the communication terminal device to specify at least one of a process of extracting the specimen from the work line and an extraction area of extracting the specimen, and obtain the specified information as the culture medium information.

7. The culture medium information registration system according to claim 1,
wherein the line related information includes at least one of line information for specifying the work line, process information for specifying the process of extracting the specimen, lot information for specifying a lot including the specimen, a specimen type indicating a type of the specimen, a culture start time of the specimen, and a dilution rate for culturing the specimen in the culture medium.

8. The culture medium information registration system according to any one of claims 1 to 7,
wherein the culture medium identification information obtaining unit obtains the culture medium information with purpose type information indicating a type of intended use of the culture medium, and
wherein the registration unit registers the culture medium information including the obtained purpose type information with the database.

9. A program executed by a computer which cooperates with a communication terminal device to register, with a database, culture medium information indicating information of a culture medium on which food produced or inspected by a work line including one or more production process or inspection process is cultured as a specimen, the program making the computer function as:
a culture medium identification information obtaining unit which cooperates with the communication terminal device to obtain culture medium identification information for identification from other culture media;
a work identification information obtaining unit which obtains work identification information which is inputted by the communication terminal device and which identifies a work of the work line;
a search unit which searches line related information related to the work line based on the obtained work identification information, the work related information including information of sanitary management of the work line stored in the database for each work identification information; and
a registration unit which registers the culture medium information including at least the obtained culture medium identification information, with the database, in correspondence with the searched line related information.

10. A communication terminal device which cooperates with a server system to register, with a database, culture medium information indicating information of a culture medium on which food produced or inspected by a work line including one or more production process or inspection process is cultured as a specimen, the communication terminal device comprising:
an image data obtaining unit which obtains image data of the specimen;
a culture medium identification information input unit which is used to input culture medium identification information for identifying a culture medium on which the specimen imaged is cultured;
a work identification information input unit which is used to input work identification information for identifying a work of the work line; and
a communication control unit which executes data communication with the server system and which registers the culture medium information including at least the obtained image data and the culture medium identification information with the database in correspondence with line related information related to the work line and including information of sanitary management of the work line stored in the database for each work identification information .

11. The communication terminal device according to claim 10,
wherein the image data obtaining unit and the culture medium information input unit are constituted by an imaging unit formed by an imaging element.

12. The communication terminal device according to claim 10 or 11, further comprising a timer which manages at least a current time,
wherein the image data obtaining unit obtains a time of obtaining the image data as an obtained time information.

13. The communication terminal device according to any one of claims 10 to 12, further comprising a process information obtaining unit which obtains process information for specifying at least one of a process of extracting the specimen from the work line and an extraction area of extracting the specimen, and
wherein the communication control unit transmits the culture medium information including the obtained process information to the server system.

14. The communication terminal device according to any one of claims 10 to 13, further comprising a detection unit which detects a number of colonies in the specimen included in the image data based on the obtained image data,
wherein the communication control unit transmits the culture medium information including the detected number of colonies to the server system.

15. The communication terminal device according to any one of claims 10 to 13, further comprising a purpose type information obtaining unit which obtains purpose type information indicating a type of intended use of the culture medium,
wherein the communication control unit transmits the culture medium information including the obtained purpose type information to the server system.

16. A program executed by a computer which cooperates with a server system to register, with a database, culture medium information indicating information of a culture medium on which food produced or inspected by a work line including one or more production process or inspection process is cultured as a specimen, the program making the computer function as:
an image data obtaining unit which obtains image data of the specimen;
a culture medium identification information input unit which is used to control an input of culture medium identification information for identifying a culture medium on which the imaged specimen is being cultured;
a work identification information input unit which is used to control an input of work identification information for identifying a work of the work line; and
a communication control unit which executes data communication with the server system and which registers the culture medium information including at least the obtained image data and the culture medium identification information with the database in correspondence with line related information related to the work line and including information of sanitary management of the work line stored in the database for each work identification information.

17. A sanitary management system comprising a communication terminal device; and a server device which cooperates with the communication terminal device to register, with a database, culture medium information indicating information of a culture medium on which food produced or inspected by a work line including one or more production process or inspection process is cultured as a specimen,
wherein the communication terminal device comprising:
an image data obtaining unit which obtains image data of the specimen;
a culture medium identification information input unit which is used to input culture medium identification information for identifying a culture medium on which the imaged specimen is cultured;
a work identification information input unit which is used to input work identification information for identifying a work of the work line; and
a communication control unit which executes data communication with the server device and which registers the culture medium information including at least the obtained image data and the culture medium identification information with the database in correspondence with line related information related to the work line and including information of sanitary management of the work line stored in the database for each work identification information, and
wherein the server device comprising:
a culture medium identification information obtaining unit which cooperates with the communication terminal device to obtain the culture medium identification information;
a work identification information obtaining unit which obtains work identification information inputted by the communication terminal device;
a search unit which searches the line related information stored in the database for each work identification information; and
a registration unit which registers the culture medium information including at least the obtained culture medium identification information, with the database, in correspondence with the searched line related information.

18. A film-type culture medium comprising:
a base material formed by a film;
a culture layer which is provided on the base material and on which food produced or inspected by a work line including one or more production process or inspection process is cultured as a specimen; and
a mark forming part on which identification information is formed in a manner readable when the identification information is imaged by a communication terminal device, the identification information being unique information used to register and manage information related to at least one of the specimen and a condition of the specimen with the database as culture medium information.
